# EUROPÄISCHE PATENTSCHRIFT

(11) **EP 0 876 344 B1**
(45) Veröffentlichungstag und Bekanntmachung des Hinweises auf die Patenterteilung: **23.08.2000**
(21) Anmeldenummer: 96937262.2
(22) Anmeldetag: 28.10.1996
(51) Int. Cl.: C07D 213/54, A01N 43/40

(54) **PYRIDYLESSIGSÄUREDERIVATE, VERFAHREN UND ZWISCHENPRODUKTE ZU IHRER HERSTELLUNG UND SIE ENTHALTENDE MITTEL**
PYRIDYLACETIC ACID DERIVATIVES, METHOD AND INTERMEDIATE PRODUCTS FOR PRODUCING THEM AND AGENTS CONTAINING THEM
DERIVES D'ACIDE ACETIQUE PYRIDYLIQUE, PROCEDE DE PREPARATION CORRESPONDANT, PRODUITS INTERMEDIAIRES UTILISES ET SUBSTANCES LES CONTENANT

(30) Priorität: 03.11.1995 DE 19540989
(43) Veröffentlichungstag der Anmeldung: 11.11.1998
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); BAYER, Herbert, D-68159 Mannheim (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); OBERDORF, Klaus, D-69117 Heidelberg (DE); RÖHL, Franz, D-67105 Schifferstadt (DE); GÖTZ, Norbert, D-67547 Worms (DE); RACK, Michael, D-69123 Heidelberg (DE); MÜLLER, Ruth, D-67159 Friedelsheim (DE); LORENZ, Gisela, D-67434 Hambach (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(86) Internationale Anmeldenummer: EP9604676
(87) Internationale Veröffentlichungsnummer: WO9717328

(56) Entgegenhaltungen:
- WO-A-95/18789
- WO-A-95/21153
- WO-A-95/21154
- WO-A-96/11183
- CHEMISCHE BERICHTE, Bd. 89, 1956, WEINHEIM DE, Seiten 1515-1521, XP002023974 W. MATHES ET AL.: "Über einige Beobachtungen in der Gruppe der Pyridinaldehyde."
- JOURNAL OF ORGANIC CHEMISTRY, Bd. 36, Nr. 20, 1971, EASTON US, Seiten 2978-2985, XP002023975 T. SASAKI ET AL.: "The facile isomerization in the 1,3-dipolar addition reactions of substituted 1-alkoxycarbonyliminopyridinium ylides with dimethyl acetylenedicarboxylate"

## Beschreibung

Die vorliegende Erfindung betrifft Pyridylessigsäurederivate der Formel I 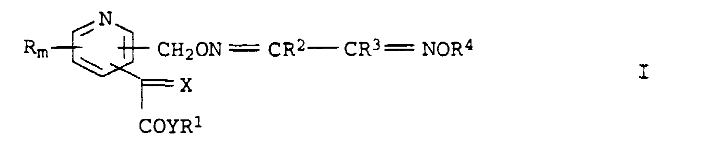 in der die Substituenten und der Index die folgende Bedeutung haben:
- X: NOCH₃, CHOCH₃, CHCH₃ und CHCH₂CH₃;
- Y: Sauerstoff oder NR';
- R': Wasserstoff oder C₁-C₄-Alkyl;
- R: Cyano, Nitro, Trifluormethyl, Halogen, C₁-C₄-Alkyl und C₁-C₄-Alkoxy;
- m: 0, 1 oder 2, wobei die Reste R verschieden sein können, wenn m für 2 steht;

- R¹: Wasserstoff und C₁-C₄-Alkyl;
- R²: Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogen-alkoxy, C₁-C₄-Alkylthio, C₁-C₄-Alkylamino oder Di-C₁-C₄-alkyl-amino;
- R³: Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR^{a})-Aₙ-R^{b};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{a})-Aₙ-R^{b} oder NR^{f}-CO-D-R^{g};
- R⁴: Wasserstoff,
C₁-C₁₀-Alkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR^{a}) -Aₙ-R^{b};
C₃-C₆-Cycloalkyl, Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{a})-Aₙ-R^{b} oder NR^{f}-CO-D-R^{g};
wobei
- A: für Sauerstoff, Schwefel oder Stickstoff steht und wobei der Stickstoff Wasserstoff oder C₁-C₆-Alkyl trägt;
- D: eine direkte Bindung, Sauerstoff oder NR^{h} bedeutet;
- n: 0 oder 1 bedeutet;
- R^{a}: Wasserstoff oder C₁-C₆-Alkyl bedeutet und
- R^{b}: Wasserstoff oder C₁-C₆-Alkyl bedeutet,
- R^{f}: Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl bedeutet;
- R^{g}, R^{h}: unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl bedeuten;
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Phenylessigsäurederivate zur Schädlingsbekämpfung bekannt (EP-A 422 597; EP-A 463 488; EP-A 370 629; EP-A 460 575; EP-A 472 300; WO-A 90/07,493; WO-A 92/13,830; WO-A 92/18,487; WO-A 95/18,789; WO-A 95/21,153; WO-A 95/21,154; WO-A 95/21,156).

Der vorliegenden Erfindung lagen neue Verbindungen mit verbesserter Wirkung als Aufgabe zugrunde.

Demgemäß wurden die eingangs definierten Pyridylessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(X)-CO₂R¹ oder die Gruppierung -CH₂ON=C(R²)-C(R³)=NOR⁴ aufgebaut wird.

Der Aufbau der Gruppierung -C(X)-CO₂R¹ ist beispielsweise aus der eingangs zitierten Literatur bekannt.

Die Art der Synthese der -CH₂ON=C(R²)-C(R³)=NOR⁴ Seitenkette richtet sich im wesentlichen nach der Art der Substituenten R² und R³.
1. Für den Fall, daß R² und R³ nicht Halogen bedeuten, geht man beim Aufbau der Gruppierung -CH₂ON=C(R²)-C(R³)=NOR⁴ im allgemeinen so vor, daß man ein Benzylderivat der Formel II mit einem Hydroxyimim der Formel III umsetzt. 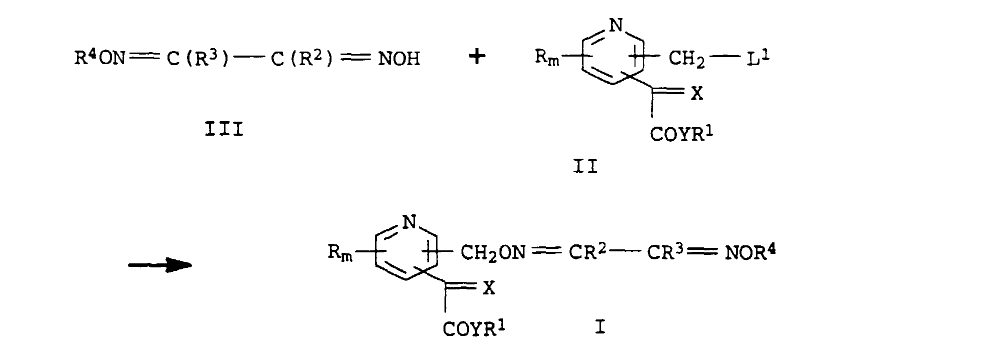
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370f und Houben-Weyl, Bd. 10/1, S. 1189f beschriebenen Methoden.
   Das benötigte Hydroxyimim III erhält man beispielsweise durch Umsetzung eines entsprechenden Dihydroxyimins IV mit einem nukleophil substituierten Reagens VI 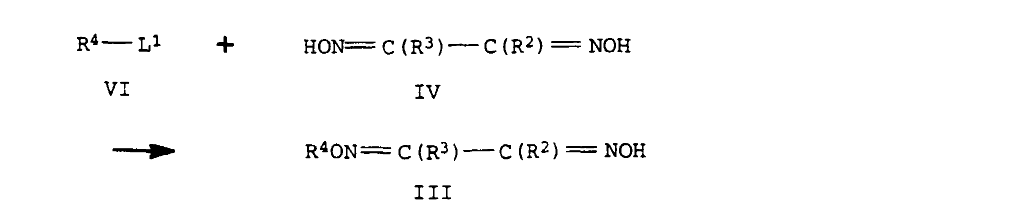
   L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
   1.1 Alternativ können die Verbindungen I auch dadurch erhalten werden, daß das Benzylderivat II zunächst mit dem Dihydroxyiminoderivat IV in ein entsprechendes Benzyloxim der Formel V umgesetzt wird, wobei V anschließend mit dem nukleophil substituierten Reagens VI zu I umgesetzt wird. 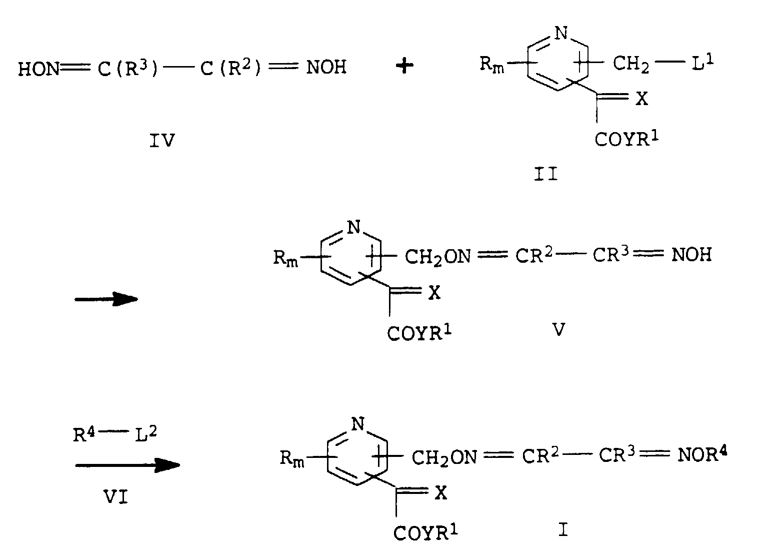
   Die Umsetzung erfolgt in an sich bekannter Weise in einem inerten organischen Lösungsmittel in Gegenwart einer Base z.B. Kaliumcarbonat, Kaliumhydroxid, Natriumhydrid, Pyridin und Triethylamin gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
   1.2 Analog ist es ebenfalls möglich, das benötigte Hydroxyimim der Formel III aus einem Carbonylhydroxyimin VII durch Umsetzung mit einem Hydroxylamin IXa oder seinem Salz IXb herzustellen. 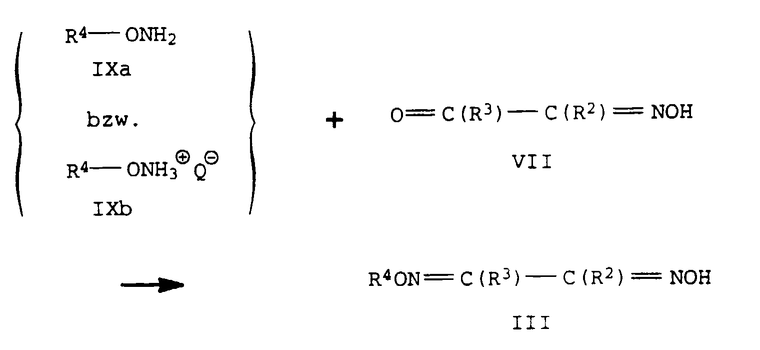
   Q^{⊖} in der Formel IXb steht für das Anion einer Säure, insbesondere einer anorganischen Säure, z.B. Halogenid wie Chlorid.
   Die Umsetzung erfolgt in an sich bekannter Weise in einem inerten organischen Lösungsmittel gemäß den in EP-A 513 580; Houben-Weyl, Bd. 10/4, S. 73f; Houben-Weyl, Bd. E 14b, S. 369f und S. 385f beschriebenen Methoden.
   1.3 Alternativ können die Verbindungen I auch dadurch erhalten werden, daß das Benzylderivat II zunächst mit dem Carbonylhydroxyiminoderivat VII in ein entsprechendes Benzyloxyimin der Formel VIII umgesetzt wird, wobei VIII anschließend mit dem Hydroxylamin IXa bzw. dessen Salz IXb zu I umgesetzt wird. 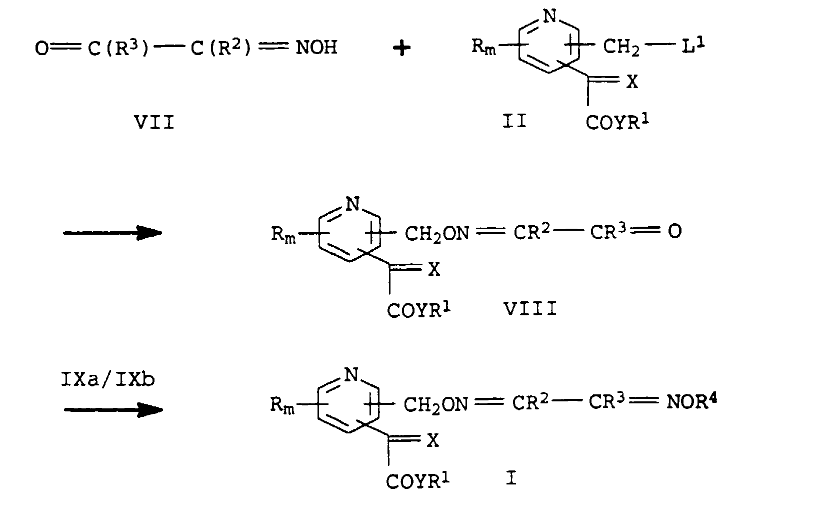
   Die Umsetzung erfolgt in an sich bekannter Weise in einem inerten organischen Lösungsmittel gemäß den in Houben-Weyl, Bd. E 14b, S. 369f; Houben-Weyl, Bd. 10/1, S. 1189f und Houben-Weyl, Bd. 10/4, S. 73f oder EP-A 513 580 beschriebenen Methoden.
   1.4 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung VIIa. 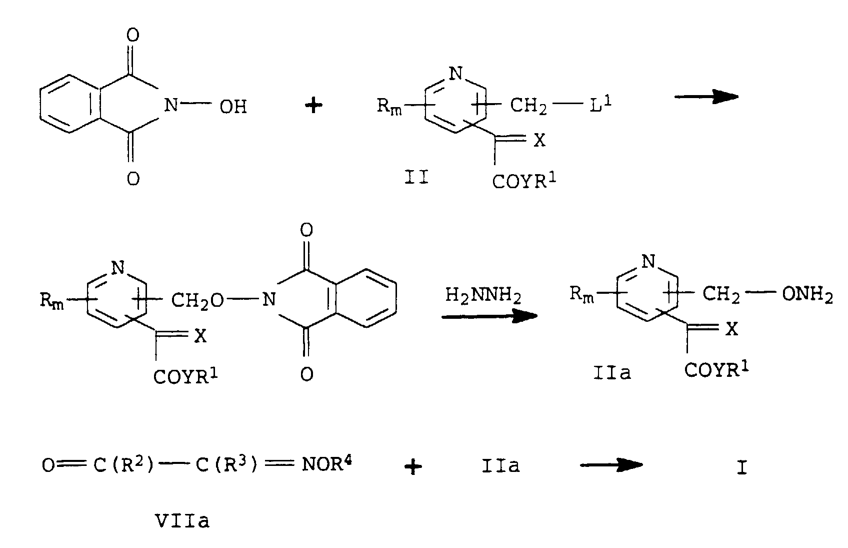
   Die Umsetzung erfolgt in an sich bekannter Weise in einem inerten organischen Lösungsmittel gemäß den in EP-A 463 488, DE-Anm. Nr. 42 28 867.3 beschriebenen Methoden.
   Die benötigte Carbonylverbindung VIIa erhält man beispielsweise durch Umsetzung einer entsprechenden Hydroxyiminocarbonylverbindung VII mit einem nukleophil substituierten Reagens VI 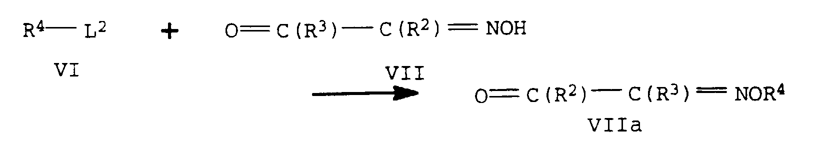 oder durch Umsetzung einer entsprechenden Dicarbonylverbindung VIIb mit einem Hydroxylamin IXa oder dessen Salz IXb 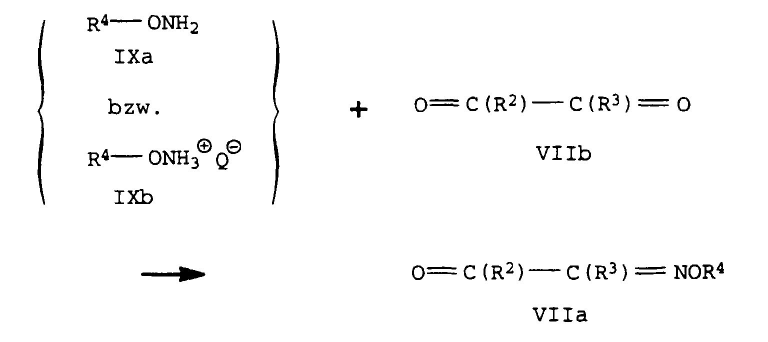
   Die Umsetzungen erfolgen in an sich bekannter Weise in einem inerten organischen Lösungsmittel gemäß den in EP-A 513 580, Houben-Weyl, Bd. 10/4, S. 55f, S. 73f, S. 180f und S. 217f; Houben-Weyl, Bd. E 14b, S. 307f und 369f, Houben-Weyl, Bd. E 5, S. 780f beschriebenen Methoden.
   1.5 Entsprechend können die Verbindungen I auch dadurch erhalten werden, daß das Benzylhydroxylamin IIa zunächst mit dem Hydroxyiminoderivat VII in das entsprechende Benzyloxyiminoderivat der Formel V umgesetzt wird, wobei V anschließend mit dem nukleophil substituierten Reagens VI wie vorstehend beschrieben zu I umgesetzt wird. 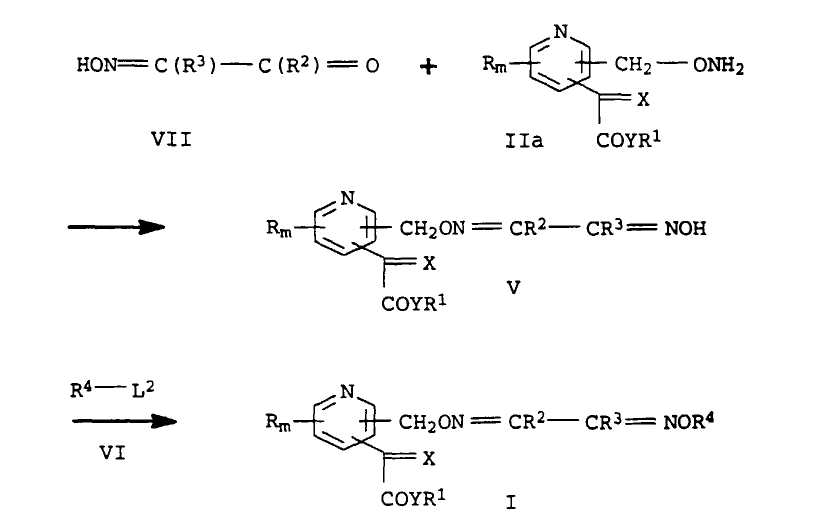
   1.6 Analog können die Verbindungen I ebenfalls dadurch hergestellt werden, daß das Benzylhydroxylamin IIa zunächst mit dem Dicarbonylderivat der Formel VIIb in das Benzyloxyiminoderivat der Formel VIII überführt wird und VIII anschließend mit dem Hydroxylamin IXa oder dessen Salz IXb wie vorstehend beschrieben zu I umgesetzt wird.

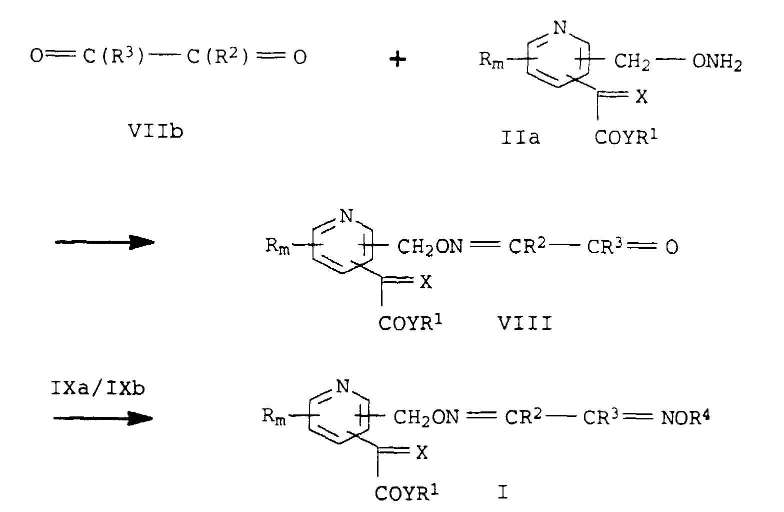

2. Verbindungen, in denen R² und/oder R³ für ein Halogenatom stehen, erhält man aus den entsprechenden Vorstufen, in denen der betreffende Rest für eine Hydroxygruppe steht nach an sich bekannten Methoden (vgl. Houben-Weyl, Vol. E5, S. 631; J. Org. Chem. 36, 233 (1971); J. Org. Chem. 57, 3245 (1992)). Vorzugsweise werden die entsprechenden Umsetzungen zum Halogenderivat auf den Stufen I und VIII vorgenommen.
3. Verbindungen, in denen R² und/oder R³ über ein O-, S- oder N-Atom an das Molekülgerüst gebunden sind, erhält man aus den entsprechenden Vorstufen, in denen der betreffende Rest für ein Halogenatom steht nach an sich bekannten Methoden (vgl. Houben-Weyl, Bd. E5, S. 826 f und 1280 f, J. Org. Chem. 36, 233 (1971), J. Org. Chem. 46, 3623 (1981)). Vorzugsweise werden die entsprechenden Umsetzungen der Halogenderivat auf den Stufen I und VIII vorgenommen.
4. Verbindungen, in denen R² und/oder R³ über ein Sauerstoffatom an das Molekül gebunden sind, erhält man zum Teil auch aus den entsprechenden Vorstufen, in denen der betreffende Rest für eine Hydroxygruppe steht nach an sich bekannten Methoden (vgl. Houben-Weyl, Bd. E5, S. 826-829, Aust. J. Chem. 27, 1341-9 (1974)). Vorzugsweise werden die entsprechenden Umsetzungen zu den Alkoxyderivaten auf den Stufen I und VIII vorgenommen.
5. Nach einem weiteren Verfahren erhält man die Verbindungen I, in denen Y für Sauerstoff steht (IA), auch dadurch, daß man einen Methylpyridincarbonsäureester der allgemeinen Formel X in an sich bekannter Weise in den entsprechenden Methylenpyridincarbonsäureester der allgemeinen Formel XI, worin Hal für Chlor oder Brom steht, überführt, XI anschließend durch Umsetzung mit einem Hydroxyimin der Formel III in den entsprechenden Pyridincarbonsäureester der allgemeinen Formel XII überführt, XII zum Alkohol XIII reduziert, XIII zum Pyridinaldehyd XIV oxidiert, XIV in an sich bekannter Weise in das Cyanhydrin XV überführt, XV anschließend zum entsprechenden Mandelsäureester XVI hydrolysiert, XVI zum α-Ketoester XVII oxidiert und XVII anschließend in an sich bekannter Weise entweder
   a) mit O-Methylhydroxylamin (H₂NOCH₃) oder einem O-Methylhydroxylammoniumsalz, oder
   b) mit einem Ethylen-Wittig- oder -Wittig-Horner Reagens, oder
   c) mit einem Methoxy-Wittig- oder -Wittig-Horner-Reagens in den entsprechenden Pyridylessigsäureester IA überführt. 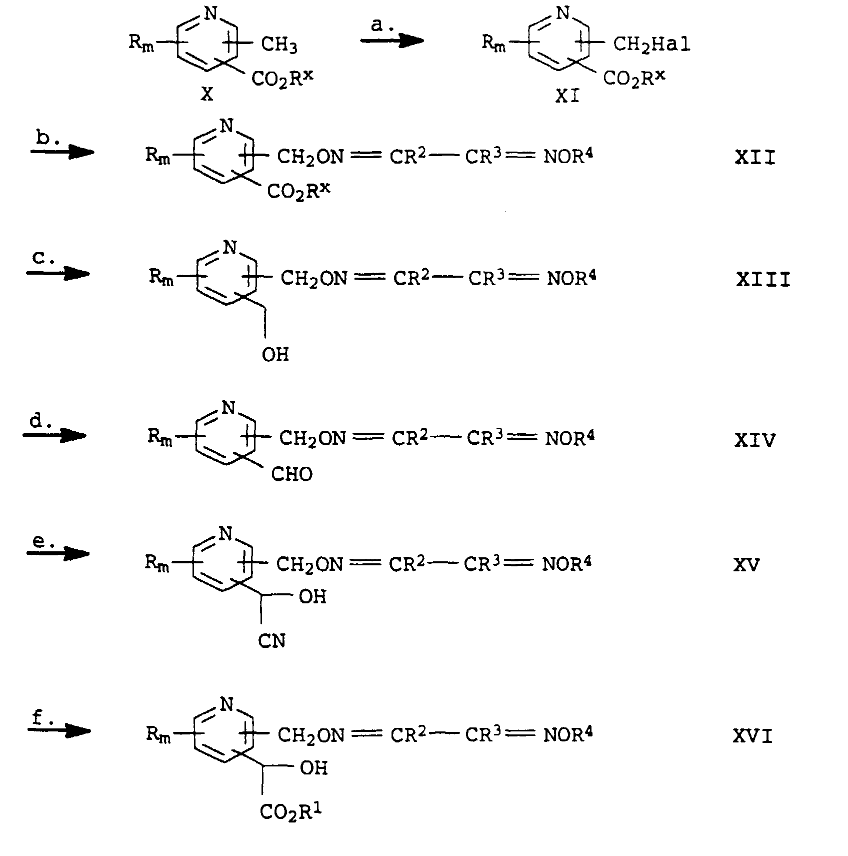 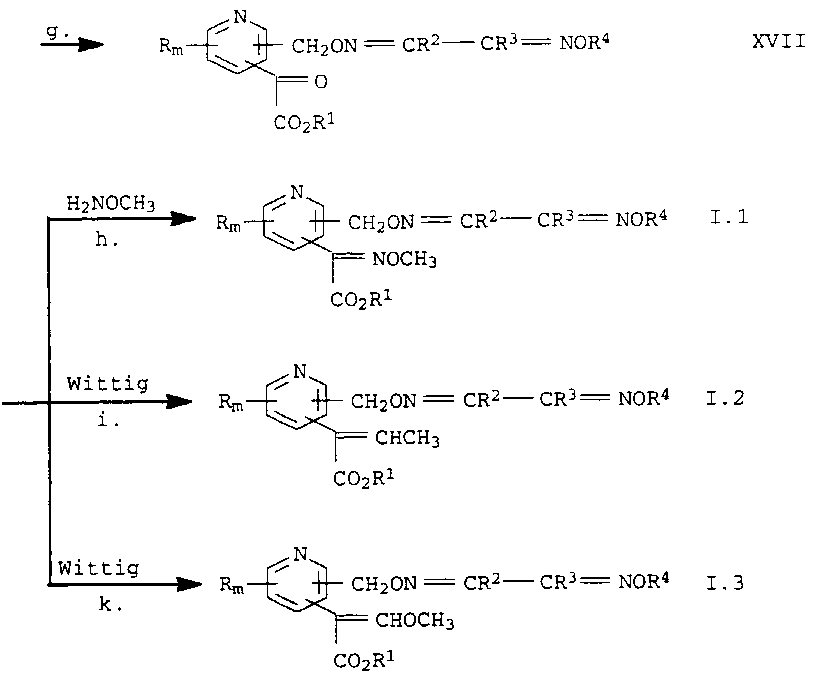
R^{x} in der Formel X steht fürC₁-C₄-Alkyl, insbesondere Methyl und Ethyl.
   Hal in der Formel XI steht für ein Halogenatom wie Fluor, Chlor, Brom und Iod, vorzugsweise Chlor und Brom, insbesondere Brom.
   5a. Diese Halogenierung von X nach XI erfolgt üblicherweise bei Temperaturen von 20°C bis 160°C, vorzugsweise 40°C bis 130°C in einem inerten organischen Lösungsmittel mit einem Halogenierungsmittel in Gegenwart eines Radikalstarters oder unter Bestrahlung mit energiereichem Licht *[vgl. Organikum, 15. Aufl. 1976, VEB Berlin, S. 196].*
      Geeignete **Lösungsmittel** sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Chlorbenzol, Methylenchlorid, Tetrachlorkohlenstoff und Cyclohexan. Es können auch Gemische der genannten Lösungsmittel verwendet werden.
      Als **Halogenierungsmittel** finden allgemein anorganische Verbindungen wie Chlor, Brom und Sulfurylchlorid Verwendung. Besonders geeignet sind aber auch organische Verbindungen wie N-Halogenide, insbesondere N-Bromsuccinimid.
      Die Halogenierungsmittel werden im allgemeinen äquimolar oder im Überschuß oder gegebenenfalls als Lösungsmittel verwendet werden.
      Es kann vorteilhaft sein, die Halogenierung in Gegenwart einer Base durchzuführen. Als **Basen** kommen allgemein anorganische Verbindungen wie Alkalimetall- und Erdalkalimetallhydroxide wie Lithiumhydroxid, Natriumhydroxid, Kaliumhydroxid und Kalziumhydroxid, Alkalimetall- und Erdalkalimetalloxide wie Lithiumoxid, Natriumoxid, Kalziumoxid und Magnesiumoxid, Alkalimetall- und Erdalkalimetallhydride wie Lithiumhydrid, Natriumhydrid, Kaliumhydrid und Kalziumhydrid, Alkalimetallamide wie Lithiumamid, Natriumamid und Kaliumamid, Alkalimetall- und Erdalkalimetallcarbonate wie Lithiumcarbonat und Kalziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxyd, Kaliumhydroxyd und Pyridin.
      Die Basen werden im allgemeinen in katalytischen Mengen ei gesetzt, sie können aber auch äquimolar, im Überschuß oder gegebenenfalls als Lösungsmittel verwendet werden.
      Als Radikalstarter finden allgemein Peroxide oder Azoverbindungen Verwendung, besonders bevorzugt Dibenzoylperoxid und Azobisisobutyronitril. Die Radikalstarter werden üblicherweise in katalytischen Mengen verwendet. Im allgemeinen sind Überschüsse an Radikalstarter nicht störend.
      Als energiereiches Licht kann in Abhängigkeit vom verwendeten Halogenierungsmittel UV-Licht oder Licht von einer Wellenlänge im sichtbaren Bereich Verwendung finden.
      Die für die Herstellung der Verbindungen I benötigten Ausgangsstoffe II können analog dem in Punkt 5. beschriebenen Verfahren ausgehend von den Nicotinsäureestern der Formel X hergestellt werden, wobei vorzugsweise zuerst der Aufbau der Gruppierung -C(=X)-COYR¹ und anschließend die Halogenierung erfolgt. 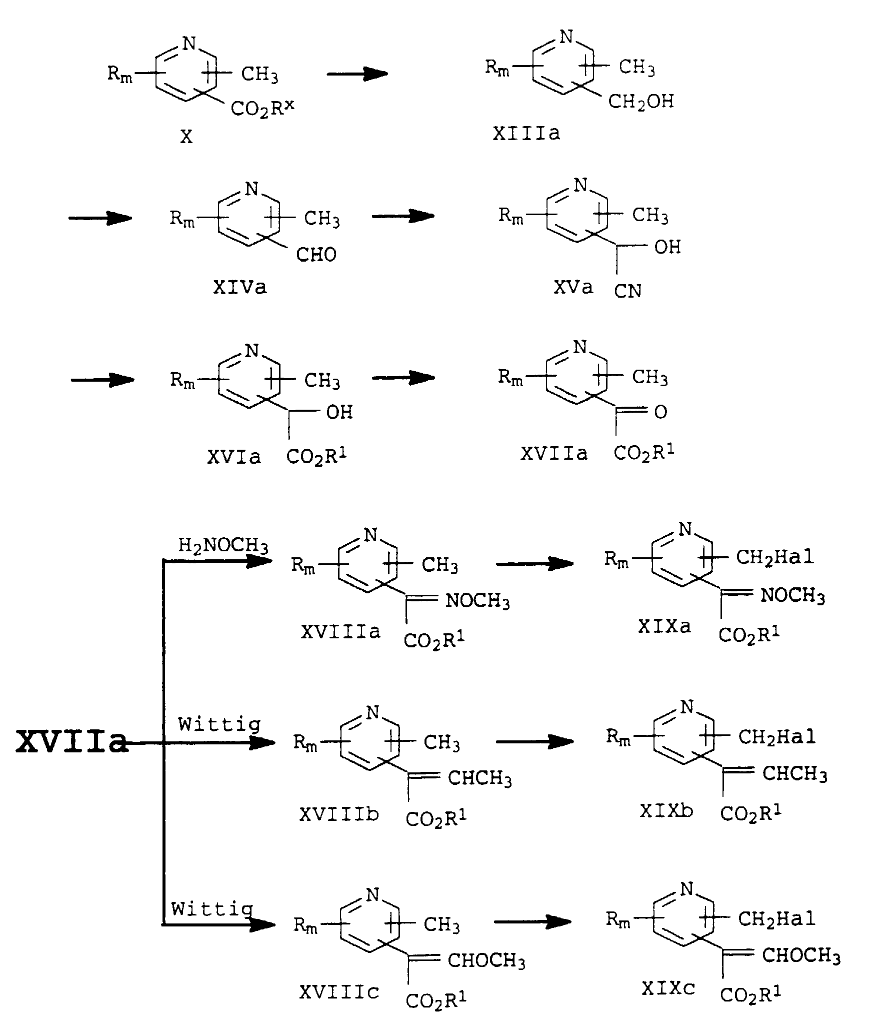
      Außerdem können die Verbindungen II aus den Verbindungen I hergestellt werden, in denen die Gruppierung -ON=CR²-CR³=NOR⁴ nach der Methode der Benzyletherspaltung [*vgl. T.* Greene,
      *Protective Groups in Organic Synthesis, J. Wiley & Sons, 1981*] abgespalten wird (z.B. mit HBr, HCl oder BBr₃).
      Desweiteren können die Verbindungen II nach dem in Punkt 5. beschriebenen Verfahren hergestellt werden, indem man bei diesem Verfahren von Derivaten ausgeht, bei denen die Seitenkette durch eine Alkoxy-, Aryloxy- oder Acyloxy-Schutzgruppe geschützt ist, und die Schutzgruppe anschließend nach literaturbekannten Methoden [*vgl. T. Greene, Protective Groups in Organic Synthesis, J. Wiley & Sons, 1981*] im letzten Verfahrensschritt abspaltet.
   5b. Diese Umsetzung von XI mit III nach XII erfolgt üblicherweise bei Temperaturen von 0°C bis 100°C, vorzugsweise 20°C bis 60°C in einem inerten organischen Lösungsmittel in Gegenwart einer Base.
      Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Dimethylformamid, Acetonitril, tert.-Butylmethylether, Toluol und Wasser. Es können auch Gemische der genannten Lösungsmittel verwendet werden.
      Als Basen kommen allgemein anorganische Verbindungen wie Alkalimetall- und Erdalkalimetallhydroxide wie Lithiumhydroxid, Natriumhydroxid, Kaliumhydroxid und Kalziumhydroxid, Alkalimetall- und Erdalkalimetalloxide wie Lithiumoxid, Natriumoxid, Kalziumoxid und Magnesiumoxid, Alkalimetall- und Erdalkalimetallhydride wie Lithiumhydrid, Natriumhydrid, Kaliumhydrid und Kalziumhydrid, Alkalimetallamide wie Lithiumamid, Natriumamid und Kaliumamid, Alkalimetall- und Erdalkalimetallcarbonate wie Lithiumcarbonat und Kalziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Kaliumcarbonat, Natriummethanolat und Natriumethanolat.
      Die Basen werden im allgemeinen in katalytischen Mengen ei gesetzt, sie können aber auch äquimolar, im Überschuß oder gegebenenfalls als Lösungsmittel verwendet werden.
   5c. Die Reduktion von XII nach XIII erfolgt üblicherweise bei Temperaturen von -70°C bis 110°C, vorzugsweise 0°C bis 60°C in einem inerten organischen Lösungsmittel mit einem Reduktionsmittel.
      Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol, Tetrahydrofuran und tert.-Butylmethylether. Es können auch Gemische der genannten Lösungsmittel verwendet werden.
      Als Reduktionsmittel finden allgemein Metallhydride Verwendung. Besonders geeignet sind Lithiumaluminiumhydrid und Natriumborhydrid. Die Reduktionsmittel können im allgemeinen äquimolar oder im Überschuß verwendet werden.
   5d. Die Oxidation von XIII nach XIV erfolgt üblicherweise bei Temperaturen von 0°C bis 60°C, vorzugsweise 10°C bis 40°C in einem inerten organischen Lösungsmittel mit einem üblichen Oxidationsmittel [*vgl. Organikum, 15. Aufl. 1976, S. 443-447, 604-607, VEB, Berlin*], besonders bevorzugt mit Natriumhypochlorit-Lösung in Gegenwart von Tetramethylpyridin-1-oxid.
      Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Methylenchlorid, Toluol, tert.-Butylmethylether und Wasser. Es können auch Gemische der genannten Lösungsmittel verwendet werden.
   5e. Diese Umsetzung von XIV nach XV erfolgt üblicherweise bei Temperaturen von -10°C bis 50°C, vorzugsweise 0°C bis 30°C in einem inerten organischen Lösungsmittel mit einem Cyanid in Gegenwart einer Säure.
      Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Diethylether, Toluol, tert.-Butylmethylether und Wasser. Es können auch Gemische der genannten Lösungsmittel verwendet werden.
      Als Cyanide finden allgemein anorganische Verbindungen Verwendung. Besonders geeignet sind Natriumcyanid oder Kaliumcyanid.
      Die Cyanide können im allgemeinen mindestens äquimolar meist sogar im Überschuß verwendet werden.
      Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid, Ammoniumchlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.
   5f. Die Hydrolyse von XV nach XVI erfolgt über den Imidoester XVa als Zwischenprodukt (X⁻ bedeutet ein Anion wie z.B. Halogenid oder Sulfat). 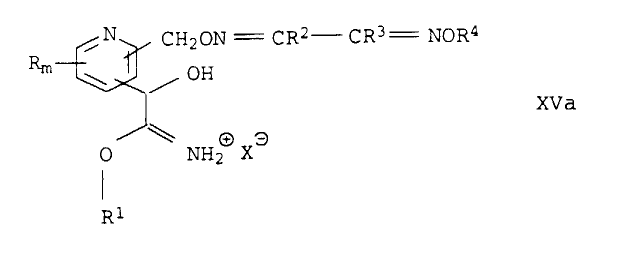
      Die Umwandlung von XV nach XVa erfolgt üblicherweise bei Temperaturen von -30°C bis 70°C, vorzugsweise bei 0°C bis 30°C in einem inerten organischen Lösungsmittel oder in dem Alkohol R¹-OH in Gegenwart einer Säure.
      Geeignete **Lösungsmittel** für die Reaktion von XV nach XVa sind aliphatische Kohlenwasserstoffe wie Pentan, HExan, Cyclohexan und Petrolether,
      aromatische Kohlenwasserstoffe wie Toluol, o-, m- und p-Xylol,
      halogenierte Kohlenwasserstoffe wie Methylenchlorid, Chloroform und Chlorbenzol,
      Ether wie Diethylether, Diisopropylether, tert.-Butylmethylether, Dioxan, Anisol und Tetrahydrofuran,
      Nitrile wie Acetonitril und Propionitril,
      Ketone wie Aceton, Methylethylketon, Diethylketon und tert.-Butylmethylketon,
      Alkohole wie Methanol, Ethanol, n-Propanol, Isopropanol, n-Butanol und tert.-Butanol sowie Dimethylsulfoxid und Dimethylformamid,
      besonders bevorzugt Diethylether, Toluol, Methanol und Ethanol.
      Es können auch Gemische der genannten Lösungsmittel verwendet werden.

      Als **Säuren** und **saure Katalysatoren** finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid,
      sowie organische Säuren wie Ameisensäure, Essigsäure, Propionsäure, Oxalsäure, Zitronensäure und Trifluoressigsäure Verwendung.
      Die Säuren werden im allgemeinen in katalytischen Mengen eingesetzt, sie können aber auch äquimolar, im Überschuß oder gegebenenfalls als Lösungsmittel verwendet werden.
      Die Hydrolyse von XV nach XVI erfolgt üblicherweise bei Temperaturen von 0°C bis 100°C, vorzugsweise 40°C bis 100°C in einem inerten organischen Lösungsmittel in Gegenwart einer Säure.
      Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Wasser, Methanol, Toluol und Acetonitril. Es können auch Gemische der genannten Lösungsmittel verwendet werden.
      Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.
      Die Säuren werden im allgemeinen in katalytischen Mengen eingesetzt, sie können aber auch äquimolar, im Überschuß oder gegebenenfalls als Lösungsmittel verwendet werden.
      Nach einer besonders bevorzugten Ausführungsform hydrolysiert man das Imidoester-hydrohalogenid in Wasser ohne weiteren Zusatz von Säure.
   5g. Diese Oxidation von XVI nach XVII erfolgt üblicherweise nach den bei Punkt 5d. genannten Verfahren.
   5h. Die Umsetzung des α-Ketoesters XVII mit O-Methylhydroxylamin (H₂NOCH₃) oder einem O-Methylhydroxylammoniumsalz erfolgt üblicherweise bei Temperaturen von 0°C bis 60°C, vorzugsweise 20°C bis 40°C gemäß [*vgl. EP-A 534 216*].
   5i. Die Umsetzung des α-Ketoesters XVII mit einem Ethylen-Wittig- oder -Wittig-Horner Reagens erfolgt üblicherweise bei Temperaturen von -30°C bis 60°C, vorzugsweise 10°C bis 40°C gemäß EP-A 534 216 in einem inerten organischen Lösungsmittel in Gegenwart einer Base.
   5k. Die Umsetzung des α-Ketoesters XVII mit einem Methoxy-Wittig- oder -Wittig-Horner-Reagens erfolgt üblicherweise bei Temperaturen von -30°C bis 60°C, vorzugsweise 10°C bis 40°C gemäß EP-A 534 216 in Gegenwart einer Base.
6. Verbindungen der Formel I, in denen Y für NR^{a} steht (IB), erhält man aus den entsprechenden Verbindungen IA in an sich bekannter Weise durch Umsetzung mit einem Amin der Formel XVIII. 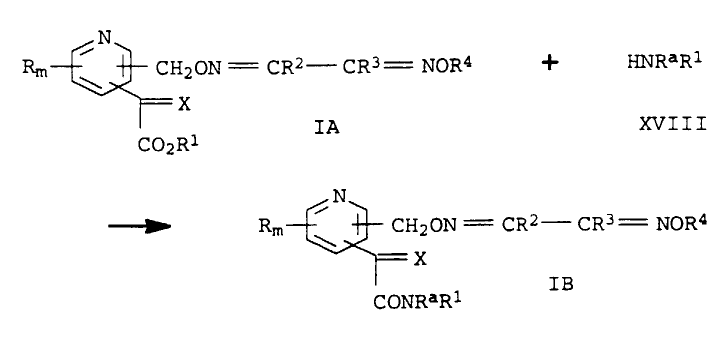
   Die Umsetzung des Esters IA mit dem Amin erfolgt üblicherweise bei Temperaturen von 0°C bis 80°C, vorzugsweise 20°C bis 60°C gemäß EP-A 534 216 in einem inerten organischen Lösungsmittel.

Die Reaktionsgemische werden in üblicher Weise aufgearbeitet, z.B. durch Mischen mit Wasser, Trennung der Phasen und gegebenenfalls chromatographische Reinigung der Rohprodukte. DieZwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=C-und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=X-Doppelbindung werden hinsichtlich ihrer Wirksamkeit die E-Isomere der Verbindungen I bevorzugt (Konfiguration bezogen auf die OCH₃ bzw. CH₃-Gruppe im Verhältnis zur COYR¹-Gruppe).

In Bezug auf die -N=CR₂-CR₃=N-Doppelbindungen werden im allgemeinen hinsichtlich ihrer Wirksamkeit die cis-Isomere der Verbindungen I (Konfiguration bezogen auf den Rest R² bzw. R³ im Verhältnis zur -OCH₂- bzw. -OR⁴-Gruppe bzw. bezogen auf den Rest R⁴ im Verhältnis zur -N=CR²-Gruppe) bevorzugt.

Bei der eingangs angegebenen Definitionen der Verbindungen I wurden Sammelbegriffe verwendet, die allgemein repräsentativ für die folgenden Gruppen stehen:
**Halogen**: Fluor, Chlor, Brom und Jod;
**Alkyl**: geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio**: geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl**: geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Cycloalkoxy bzw. Cycloalkylthio und Cycloalkylamino:** monocyclische Alkenylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy-, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,l-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino**: aromatische mono- oder polycyclische Kohlenwasserstoffreste, welche über ein Stickstoffatom an das Gerüst gebunden sind.
**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-cliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
   bzw. die entsprechenden Oxy-, Thio-, Carbonyl- oder Sulfonylgruppen.
**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen m für 0 oder 1, insbesondere 0, steht.

Für den Fall, daß m für 1 steht werden Verbindungen I bevorzugt, in denen R für Chlor oder Fluor steht.

Besonders werden Verbindungen I bevorzugt, in denen X für NOCH3 (Formel I.1) steht.

Außerdem werden Verbindungen I bevorzugt, in denen X für CHCH3 (Formel I.2) steht.

Gleichermaßen werden Verbindungen I bevorzugt, in denen X für CHOCH₃ (Formel I.3) steht.

Daneben werden Verbindungen I besonders bevorzugt, in denen R¹ für Methyl steht.

Desweiteren werden Verbindungen I bevorzugt, in denen Y für Sauerstoff steht (Formel IA).

Gleichermaßen werden Verbindungen I bevorzugt, in denen Y für NR', insbesondere NH, steht (Formel IB).

Außerdem werden Verbindungen I besonders bevorzugt, in denen R² für C₁-C₄-Alkyl, insbesondere Methyl, steht.

Gleichermaßen besonders bevorzugt sind Verbindungen I, in denen R² für Trifluormethyl steht.

Daneben werden Verbindungen I besonders bevorzugt, in denen R² für Cyclopropyl steht.

Desweiteren werden Verbindungen I besonders bevorzugt, in denen R² für C₁-C₄-Alkoxy, insbesondere Methoxy, steht.

Außerdem werden Verbindungen I besonders bevorzugt, in denen R² für Halogen, insbesondere Chlor, steht.

Insbesondere werden auch Verbindungen I bevorzugt, in denen R³ für Aryl, insbesondere ggf. subst. Phenyl, steht.

Außerdem werden Verbindungen I besonders bevorzugt, in denen R³ für Alkyl, insbesondere C₁-C₆-Alkyl, steht.

Gleichermaßen besonders bevorzugt sind Verbindungen I, in denen R³ für Alkoxy, insbesondere C₁-C₆-Alkoxy, steht.

Daneben werden Verbindungen I besonders bevorzugt, in denen R³ für Cycloalkyl, insbesondere C₃-C₆-Cycloalkyl, steht.

Desweiteren werden Verbindungen I besonders bevorzugt, in denen R³ für Aryloxyalkyl, insbesondere im Phenylteil ggf. subst. Phenoxy-C₁-C₂-alkyl, steht.

Außerdem werden Verbindungen I besonders bevorzugt, in denen R³ für Hetaryl, insbesondere ggf. subst. Thienyl, Isoxazolyl, Pyrazolyl und Pyridinyl, steht.

Insbesondere werden auch Verbindungen I bevorzugt, in denen R⁴ für C₁-C₆-Alkyl, insbesondere Methyl, steht.

Außerdem werden Verbindungen I besonders bevorzugt, in denen R⁴ für C₃-C₆-Alkenyl, insbesondere Allyl, steht.

Gleichermaßen besonders bevorzugt sind Verbindungen I, in denen R⁴ für C₃-C₆-Alkinyl, insbesondere Propargyl steht.

Daneben werden Verbindungen I besonders bevorzugt, in denen R⁴ für Arylalkyl, insbesondere im Phenylteil ggf. subst. Phenyl-C₁-C₂-alkyl, steht.

Desweiteren werden Verbindungen I besonders bevorzugt, in denen R⁴ für Haloalkenyl, insbesondere trans-Chlorallyl, steht.

Außerdem werden Verbindungen I besonders bevorzugt, in denen R⁴ für Alkoxyalkyl, insbesondere C₁-C₂-Alkoxy-C₁-C₂-alkyl, steht.

Insbesondere werden Verbindungen I bevorzugt, in denen der Rest C(=X)-COYR¹ in 2-Position des Pyridylrings gebunden ist.

Daneben werden Verbindungen I bevorzugt, in denen der Rest C(=X)-COYR¹ in 3-Position des Pyridylrings gebunden ist.

Außerdem werden Verbindungen I bevorzugt, in denen der Rest C(=X)-COYR¹ in 4-Position des Pyridylrings gebunden ist.

Insbesondere werden auch Verbindungen I bevorzugt, in denen der Rest -CH₂ON=CR²-CR³=NOR⁴ in 2-Position des Pyridylrings gebunden ist.

Daneben werden Verbindungen I bevorzugt, in denen der Rest-CH₂ON=CR²-CR³=NOR⁴ in 3-Position des Pyridylrings gebunden ist.

Außerdem werden Verbindungen I bevorzugt, in denen der Rest-CH₂ON=CR²-CR³=NOR⁴ in 4-Position des Pyridylrings gebunden ist.

Insbesondere werden Verbindungen I bevorzugt, in denen der Rest C(=X)-COYR¹ in 3-Position und der Rest -CH₂ON=CR²-CR³=NOR⁴ in 2-Position des Pyridylrings gebunden ist.

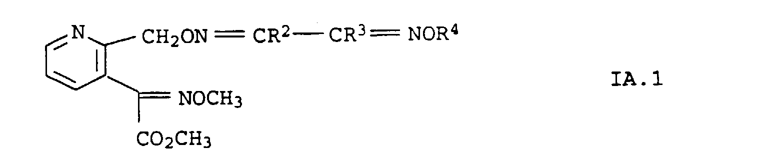

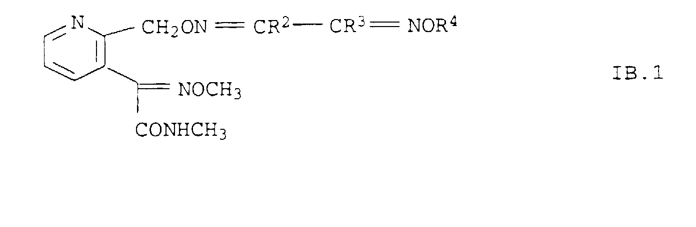

**Tabelle I:**

| Ausgewählte physikal. Daten einiger Verbindungen | | | | | |
|---|---|---|---|---|---|
| Nr. | Formel | R² | R³ | R⁴ | physikal. Daten |
| I.1 | IA.1 | CH₃ | 2,4-Cl₂-C₆H₃ | CH₃ | 111-112 |
| I.2 | IA.1 | CH₃ | C₆H₅ | CH₃ | 133-135 |
| I.3 | IA.1 | CH₃ | 4-F-C₆H₄ | CH₃ | 102-103 |
| I.4 | IA.1 | CH₃ | 4-Cl-C₆H₄ | CH₃ | 141-142 |
| I.5 | IA.1 | CH₃ | 4-i-C₃H₇-C₆H₄ | CH₃ | 121-122 |
| I.6 | IB.1 | CH₃ | 4-F-C₆H₄ | CH₃ | 120-122 |
| I.7 | IB.1 | CH₃ | 4-Cl-C₆H₄ | CH₃ | 133-135 |
| I.8 | IB.1 | CH₃ | 2,4-Cl₂-C₆H₃ | CH₃ | 160-162 |
| I.9 | IB.1 | CH₃ | 4-i-C₃H₇-C₆H₄ | CH₃ | 114-117 |
| physikal. Daten: Fp [°C] IR [cm⁻¹] oder ¹H-NMR [ppm] | | | | | |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindungen gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl-disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthal-imidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)4-n-propyl-1,3-dioxolan-2-yl-ethyl]-1H-1,2,4-triazol, N-(n-Propyl)-N- (2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methylpyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Kalziumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalzium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.
1. Synthese von 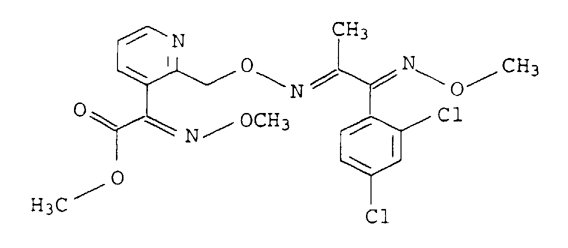
   a) 2-Methyl-3-formylpyridin-cyanhydrin
      Eine Mischung von 33 g (0,5 Mol) KCN und 26 g (0,5 Mol NH₄Cl in 200 ml Wasser und 30 g (0,24 Mol) 2-Methyl-3-formylpyridin (Chem. Pharm. Bull. 42 (1994), 1941; J. Med. Chem. 32 (1989), 583; J. Heterocycl. Chem. 28 (1991), 1315; J. Org. Chem. 43 (1978), 324) in 200 ml Diethylether wird 2 Stunden bei Raumtemperatur gerührt, wobei das Produkt auskristallisiert. Der Festkörper wird abgesaugt, mit Methyl-t-butylether gewaschen und im Stickstoffstrom getrocknet (Ausbeute: 19,3 g (54 %); Fp = 139°C).
      ¹H-NMR (DMSO-d₆; δ in ppm):
      8,5 (d, breit, 1H, Pyridyl); 7,9 (d, breit, 1H, Pyridyl); 7,3 (dd, 1H, Pyridyl; 7,2 (s, breit, 1H, OH); 5,9 (s, 1H, CH); 2,55 (s, 3H, CH₃).
   b) (2-Methylpyridyl-3)-α-hydroxy-essigsäuremethylester
      In eine Lösung von 22 g (0,15 Mol) 2-Methyl-3-formylpyridin-cyanhydrin (Beispiel la) in 250 ml Methanol werden 15 g (0,4 Mol) gasförmige Salzsäure eingeleitet. Man rührt die Reaktionsmischung über Nacht bei Raumtemperatur und dampft anschließend im Vakuum ein. Der Rückstand wird in 200 ml Wasser aufgenommen und 1 Stunde zum Rückfluß erhitzt. Anschließend wird die Reaktionsmischung auf Raumtemperatur abgekühlt, mit NaHCO₃-Lösung neutralisiert und mit Methylenchlorid extrahiert. Die vereinigten organischen Phasen werden über MgSO₄ getrocknet und eingeengt. Der Rückstand wird säulenchromatographisch mit Cyclohexan/Essigester-Gemischen gereinigt. Man erhält 3,0 g (11 %) der Titelverbindung als helles Öl.
      ¹H-NMR (CDCl₃; δ in ppm):
      8,35 (d, breit, 1H, Pyridyl); 7,7 (d, breit, 1H, Pyridyl); 7,15 (dd, 1H, Pyridyl); 5,4 (s, 1H, CH); 4,5 (s, sehr breit, 1H, OH); 3,75 (s, 3H, OCH₃); 2,6 (s, 3H, CH₃).
   c) (2-Methylpyridyl-3)-glyoxylsäuremethylester
      Eine gerührte Mischung von 3 g (16 mmol) (2-Methylpyridyl-3)-α-hydroxy-essigsäuremethylester (Beispiel 1b) in 20 ml Methylenchlorid und 0,5 g (3,5 mmol) Na₂HPO₄, 0,6 g (5 mmol) NaH₂PO₄ und 0,2 g (1,6 mmol) KBr in 20 ml Wasser wird mit einer Spatelspitze Tetramethylpyridin-N-oxyl und 10 ml 12,5 %iger Na-Hypochlorit-Lösung versetzt. Man rührt 1 Stunde bei Raumtemperatur, trennt anschließend die organische Phase ab und engt sie ein. Als Rückstand erhält man 2,2 g (77 %) der Titelverbindung als gelbes Öl.
      ¹H-NMR (CDCl₃; δ in ppm):
      8,7 (d, breit, 1H, Pyridyl), 8,05 (d, breit, 1H, Pyridyl); 7,3 (dd, 1H, Pyridyl); 4,0 (s, 3H, OCH₃); 2,8 (s, 3H, CH₃).
   d) (2-Methylpyridyl-3)-glyoxylsäuremethylester-trans-O-methyloxim
      Eine Mischung von 2,2 g (12 mmol) (2-Methylpyridyl-3)-glyoxylsäuremethylester (Beispiel lc) und 1,5 g (18 mmol) o-Methylhydroxylamin-hydrochlorid in 20 ml Methanol wird über Nacht bei Raumtemperatur gerührt. Anschließend wird die Reaktionsmischung eingeengt. Der Rückstand wird in Methylenchlorid aufgenommen und mit wenig Wasser extrahiert. Die organische Phase wird über MgSO₄ getrocknet und eingeengt. Der Rückstand wird säulenchromatographisch mit Cyclohexan-Essigester-Gemischen gereinigt. Man erhält 1,8 g (73 %) der Titelverbindung als helles Öl.
      ¹H-NMR (CDCl₃, δ in ppm):
      8,55 (d, breit, 1H, Pyridyl); 7,45 (d, breit, 1H, Pyridyl), 7,2 (dd, 1H, Pyridyl); 4,1 (s, 3H, OCH₃); 3,9 (s, 3H, OCH₃); 2,45 (s, 3H, CH₃).
   e) (2-Brommethylpyridyl-3)-glyoxylsäuremethylester-trans-O-methyloxim 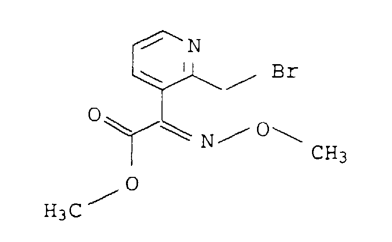 Eine Mischung von 1,8 g (8,7 mmol) (2-Methylpyridyl-3)-glyoxylsäuremethylester-trans-O-methyloxim, 1,7 g (9,6 mmol) N-Bromsuccinimid und einer Spatelspitze Azoisobutyrodinitril in 30 ml CCl₄ wird ca. 4 Stunden mit einer 300 W UV-Lampe bestrahlt, wobei sich die Reaktionsmischung auf Rückflußtemperatur erwärmt. Anschließend verdünnt man die Reaktionsmischung mit Methylenchlorid und extrahiert die organische Phase mit Wasser. Die organische Phase wird mit Wasser extrahiert, über MgSO₄ getrocknet und eingeengt. Der Rückstand wird säulenchromatographisch mit Cyclohexan/Essigester-Gemischen gereinigt. Man erhält 1,1 g (44 %) der Titelverbindung als helles Öl.
      ¹H-NMR (CDCl₃; δ in ppm):
      8,65 (d, breit, 1H, Pyridyl); 7,55 (d, breit, 1H, Pyridyl); 7,3 (t, breit, 1H, Pyridyl); 4,4 (s, 2H, CH₂Br); 4,1 (s, 3H, OCH₃); 3,9 (s, 3H, OCH₃).
   f) 2-Oximino-1-(2',4'-dichlorphenyl)-propan-1-on
      Eine Mischung von 60 g (0,296 mol) 2,4-Dichlorpropiophenon in 500 ml Toluol wird bei -10 bis -20°C mit 81 ml gesättigter etherischer Salzsäurelösung und anschließend bei der gleichen Temperatur mit einer Lösung von 33,3 g (0,323 mol) 2,4-Dichlorpropiophenon in 150 ml Ether versetzt. Anschließend rührt man die Reaktionsmischung 4 Stunden bei -10°C und 14 Stunden bei Raumtemperatur. Dann extrahiert man die Reaktionsmischung mit Eiswasser und anschließend fünfmal mit ln NaOH. Die vereinigten alkalischen Phasen werden mit 20 %iger Schwefelsäure auf pH 5 angesäuert, wobei das Produkt auskristallisiert. Der Festkörper wird abfiltriert und in Methylenchlorid gelöst und die organische Phase wird über MgSO₄ getrocknet und eingeengt. Als Rückstand erhält man 63,1 g (42 %) der Titelverbindung als hellen FEstkörper.
      ¹H-NMR (CDCl₃, δ in ppm):
      8,8 (s, 1H, OH); 7,4 (s, 1H, Phenyl); 7,3 (m, 2H, Phenyl); 2,1 (s, 3H, CH₃).
   g) 1-Methoximino-2-oximino-1-(2',4'-dichlorphenyl)-propan
      Eine Mischung von 4,7 g (20 mmol)
      2-Oximino-1-(2',4'-dichlorphenyl)-propan-1-on (Beispiel lf), 2,5 g (30 mmol) O-Methylhydroxylamin-hydrochlorid und 4,8 g (60 mmol) Pyridin wird über Nacht bei Raumtemperatur und anschließend 8 Stunden bei 50°C gerührt. Sodann wird die Reaktionsmischung eingeengt und der Rückstand wird in Methylenchlorid aufgenommen. Die organische Phase wird mit verdünnter Salzsäure und Wasser gewaschen, über MgSO₄ getrocknet und eingeengt. Der Rückstand kristallisiert und wird mit Hexan ausgerührt. Man erhält 3 g (57 %) der Titelverbindung als farblosen Festkörper.
      ¹H-NMR (CDCl₃, δ in ppm):
      8,25 (s, 1H, OH); 7,4 (s, 1H, Phenyl); 7,25 (d, breit, 1H, Phenyl); 7,0 (d, 1H, Phenyl); 3,9 (s, 3H, OCH₃); 2,15 (s, 3H, CH₃).
   h) Synthese von 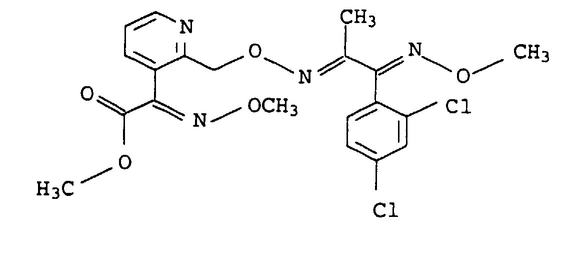 Eine Mischung von 1 g (3,8 mmol) 1-Methoximino-2-oximino-1-(2',4'-dichlorphenyl)-propan (Beispiel 1g) in 20 ml Dimethylformamid wird mit 0,12 g (5 mmol) Natriumhydrid versetzt. man rührt bis die Gasentwicklung beendet ist und gibt anschließend 1,1 g (3,8 mmol) (2-Brommethylpyridyl-3)-glyoxylsäuremethylester-trans-O-methyloxim (Beispiel le) hinzu. Man rührt eine Stunde bei Raumtemperatur, verdünnt die Reaktionsmischung anschließend mit Wasser und extrahiert die wäßrige Phase dreimal mit Methyl-t-butylether. Die vereinigten organischen Phasen werden über MgSO₄ getrocknet und eingeengt. Der Rückstand wird säulenchromatographisch mit Cyclohexan/Essigester-Gemischen gereinigt. Das Produkt kristallisiert und wird mit Hexan ausgerührt. Man erhält 0,35 g (20 %) der Titelverbindung als farblosen Festkörper (FP = 111°C).
      ¹H-NMR (CDCl₃, δ in ppm):
      8,55 (s, breit, 1H, Pyridyl); 7,55 (d, breit, 1H; Pyridyl); 7,35 (s, breit, 1H, Phenyl), 7,3 (dd, 1H, Pyridyl); 7,15 (d, breit, 1H, Phenyl); 6,9 (d, 1H, Phenyl); 5,05 (s, 2H, OCH₂); 4,05 (s, 3H, OCH₃); 3,9 (s, 3H, OCH₃); 3,85 (s, 3H, OCH₃); 2,15 (s, 3H, CH₃).

### Beispiele zur Wirkung gegen Schadpilze

Die fungizide Wirkung der Verbindungen der Formel I ließ sich durch folgende Versuche zeigen:

Die Wirkstoffe wurden als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.
A) Wirksamkeit gegen Weizenmehltau
   Blätter von in Töpfen gewachsenen Weizenkeimlingen der Sorte "Frühgold" wurden mit wäßriger Spritzbrühe, die mit einer Stammlösung aus 10 % Wirkstoff, 63 % Cyclohexanon und 27 % Emulgiermittel angesetzt wurde, besprüht und 24 Stunden nach dem Antrocknen des Spritzbelages mit Sporen des Weizenmehltaus (*Erysiphe graminis var. tritici*) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung visuell in % Befall der gesamten Blattfläche ermittelt.
   In diesem Test zeigten die mit 250 ppm der Verbindungen Nr. 1.2, I.3, I.4, I.6, I.7, I.8 und I.9 behandelten Pflanzen maximal 15 % Befall, während die unbehandelten Pflanzen zu 75 % befallen waren.
B) Wirksamkeit gegen *Plasmopara viticola*
   Blätter von Topfreben der Sorte "Müller-Thurgau" wurden mit wäßriger Spritzbrühe, die mit einer Stammlösung aus 10 % Wirkstoff, 63 % Cyclohexanon und 27 % Emulgiermittel angesetzt wurde, besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.
   In diesem Test zeigten die mit 250 ppm der Verbindungen Nr. 1, I.2, I.3, I.4, I.6 und I.9 behandelten Pflanzen maximal 15 % Befall, während die unbehandelten Pflanzen zu 70 % befallen waren.

### Beispiele zur Wirkung gegen tierische Schädlinge

Die Wirkung der Verbindungen der allgemeinen Formel I gegen tierische Schädlinge ließ sich durch folgende Versuche zeigen: Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Pyridylessigsäurederivate der Formel I 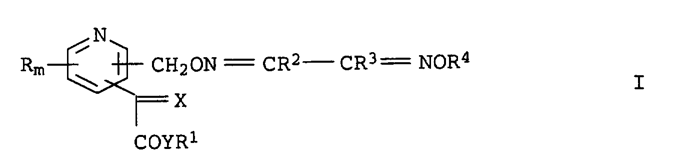 in der die Substituenten und der index die folgende Bedeutung haben:
X NOCH₃, CHOCH₃, CHCH₃ und CHCH₂CH₃;
Y Sauerstoff oder NR';
R' Wasserstoff oder C₁-C₄-Alkyl;
R Cyano, Nitro, Trifluormethyl, Halogen, C₁-C₄-Alkyl und C₁-C₄-Alkoxy;
m 0, 1 oder 2, wobei die Reste R verschieden sein können, wenn m für 2 steht;
R¹ Wasserstoff und C₁-C₄-Alkyl;
R² Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino oder Di-C₁-C₄-alkylamino;
R³ Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆,-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{a})-Aₙ-R^{b};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{a})-Aₙ-R^{b} oder NR^{f}-CO-D-R^{g};
R⁴ Wasserstoff,
C₁-C₁₀-Alkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, AryIoxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminochiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR^{a})-Aₙ-R^{b};
C₃-C₆-Cycloalkyl, Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{a})-Aₙ-R^{b} oder NR^{f}-CO-D-R^{g};
wobei
A für Sauerstoff, Schwefel oder Stickstoff steht und wobei der Stickstoff Wasserstoff oder C₁-C₆-Alkyl trägt;
D eine direkte Bindung, Sauerstoff oder NR^{h};
n 0 oder 1 bedeutet;
R^{a} Wasserstoff oder C₁-C₆-Alkyl bedeutet und
R^{b} Wasserstoff oder C₁-C₆-Alkyl bedeutet,
R^{f} Wasserstoff, Hydroxy, C₁-C₆-Alkyl, C₂-C₆-Alkenyl,
C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
R^{g}, R^{h} unabhängig voneinander Wasserstoff, C₁-C₆-Alkyl,
C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
sowie deren Salze.

2. Verbindungen der Formel I gemäß Anspruch 1, in denen R², R³ und R⁴ die folgende Bedeutung haben:
R² Wasserstoff, Hydroxy, Cyclopropyl, Halogen, C₁-C₄-Alkyl, C₁-C₄-Alkoxy oder C₁-C₄-Alkylthio;
R³ Wasserstoff, Hydroxy, Halogen, Cyclopropyl, Cyclohexyl, C₁-C₄-A1kyl, C₁-C₄-Alkoxy, C₁-C₄-Alkylthio,
Aryl oder Hetaryl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
R⁴ Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Gruppen partiell oder vollständig halogeniert sein können, oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy und Hetarylthio, wobei die aromatischen und heteroaromatischen Reste ihrerseits partiell oder vollständig halogeniert sein können und/ oder ein bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl Hetaryloxy, Hetarylthio oder C(=NOR^{a})-Aₙ-R^{b};
Aryl, Hetaryl, Arylcarbonyl, Hetarylcarbonyl, Arylsulfonyl oder Hetarylsulfonyl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR^{a})-Aₙ-R^{b}.

3. Verbindungen der Formel I gemäß Anspruch 1, in denen Y für NR' und X für NOCH₃ steht.

4. Verbindungen der Formel I gemäß Anspruch 1, in denen m für 0 steht.

5. Verbindungen der Formel I gemäß Anspruch 1, in denen R¹ für Methyl steht.

6. Verfahren zur Herstellung der Verbindungen der Formel I gemäß Anspruch 1, in denen R² nicht Halogen bedeutet, dadurch gekennzeichnet, daß man ein Benzylderivat der Formel II 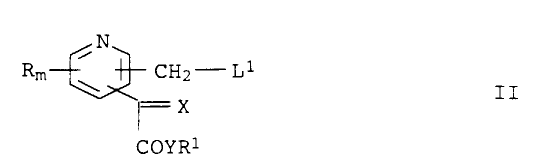 in der L¹ für eine nucleophil austauschbare Abgangsgruppe steht, in an sich bekannter Weise mit einem Hydroxyimim der Formel III
R⁴ON=C(R³)-C(R²)=NOH III
umsetzt.

7. Verfahren zur Herstellung der Verbindungen der Formel I gemäß Anspruch 1, in denen R² und R³ nicht Halogen bedeutet, dadurch gekennzeichnet, daß man ein Benzylderivat der Formel II gemäß Anspruch 1 in an sich bekannter Weise mit einem Dihydroxyimim der Formel IV
HON=C(R³)-C(R²)=NOH IV
zu einer Verbindung der Formel V 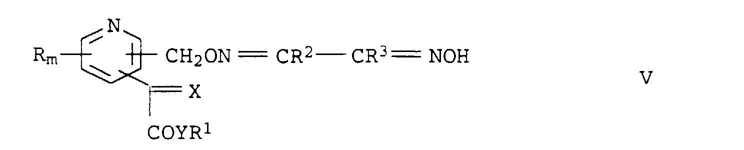 umsetzt und V anschließend mit einer Verbindung der Formel VI
R⁴-L¹ VI
in der L² für eine nucleophil austauschbare Abgangsgruppe steht, zu I umsetzt.

8. Verfahren zur Herstellung der Verbindungen der Formel I gemäß Anspruch 1, in denen R² nicht Halogen bedeuter, dadurch gekennzeichnet, daß man ein Benzylderivat der Formel II gemäß Anspruch 1 in an sich bekannter Weise mit einem Carbonylhydroxyimin der Formel VII
O=C(R³)-C(R²)=NOH VII
zu einer Verbindung der Formel VIII 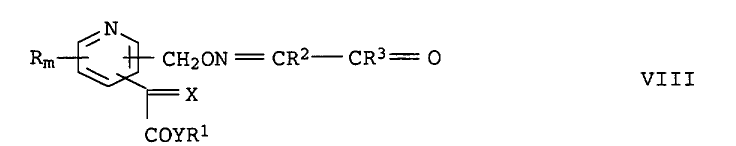 umsetzt, VIII anschließend entweder
a) zunächst mit Hydroxylamin oder dessen Salz und danach mit einer Verbindung der Formel VI (R⁴-L²) gemäß Anspruch 7 oder
b) mit einem Hydroxylamin oder einem Hydroxylammoniumsalz der Formel IXa bzw. IXb 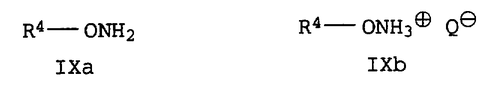 in der Q^{⊖} für das Anion einer Säure steht,
zu I umsetzt.

9. Verfahren zur Herstellung der Verbindungen I gemäß Anspruch 1, in denen Y für Sauerstoff steht (IA), dadurch gekennzeichnet, daß man einen Methylpyridincarbonsäureester der allgemeinen Formel X 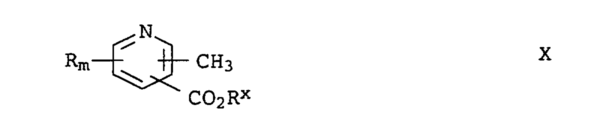 in der R^{x} für C₁-C₄-Alkyl steht, in an sich bekannter Weise in den entsprechenden Methylenpyridincarbonsäureester der allgemeinen Formel XI 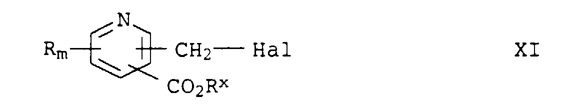 überführt, XI anschließend durch Umsetzung mit einem Hydroxyimin der Formel III gemäß Anspruch 6 in den entsprechenden Pyridincarbonsäureester der allgemeinen Formel XII 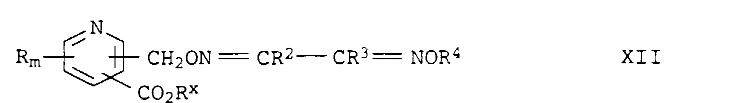 überführt, XII zum Alkohol XIII 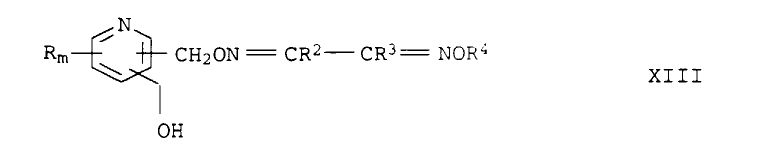 reduziert, XIII zum Pyridinaldehyd XIV 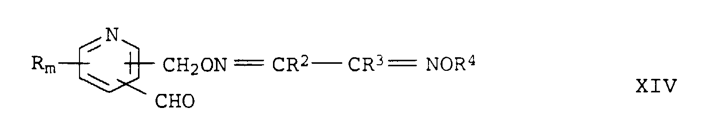 oxidiert, XIV in an sich bekannter Weise in das Cyanhydrin XV 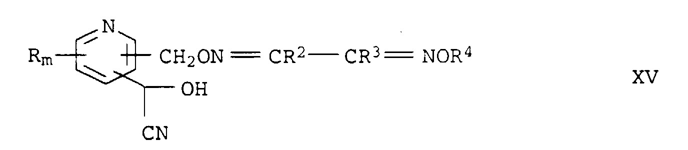 überführt, XV anschließend zum entsprechenden Mandelsäureester XVI 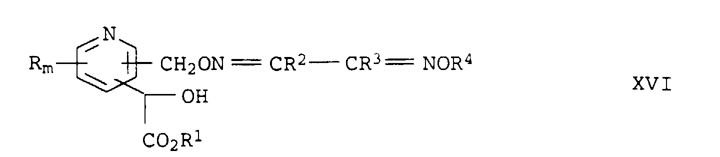 hydrolysiert, XVI zum α-Ketoester XVII 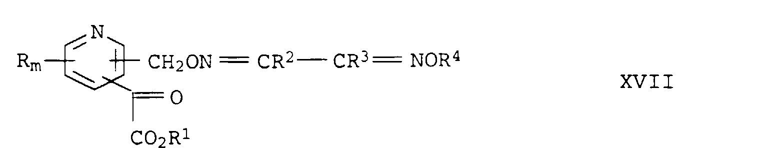 oxidiert und XVII anschließend in an sich bekannter Weise entweder
a) mit O-Methylhydroxylamin (H₂NOCH₃) oder einem O-Methylhydroxylammoniumsalz, oder
b) mit einem Ethylen-Wittig- oder -Wittig-Horner Reagens, oder
c) mit einem Methoxy-Wittig- oder -Wittig-Horner Reagens
in den entsprechenden Pyridylessigsäureester IA überführt.

10. Verfahren zur Herstellung der Verbindungen 1 gemäß Anspruch 1, in denen Y für NR' steht (IB), dadurch gekennzeichnet, daß man den entsprechenden Pyridylessigsäureester der Formel IA gemäß Anspruch 9 in an sich bekannter Weise mit einem Amin der Formel XVIII
HNR'R¹ XVIII
umsetzt.

11. Verfahren zur Herstellung der Verbindungen I gemäß Anspruch 1, dadurch gekennzeichnet, daß man ein Hydroxylaminderivat der Formel IIa 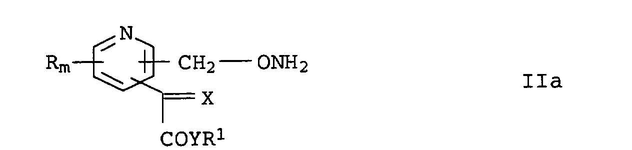 in an sich bekannter Weise mit einer Carbonylverbindung der Formel VIIa 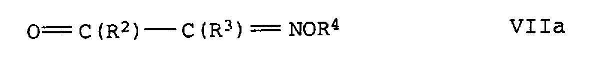 umsetzt.

12. Verfahren zur Herstellung der Verbindungen I gemäß Anspruch 1, in denen Y für Sauerstoff steht (IA), dadurch gekennzeichnet, daß man einen Methylpyridincarbonsäureester der Formel X 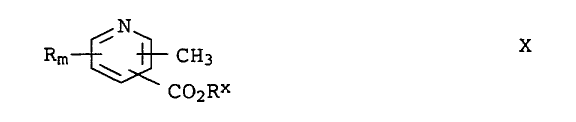 in der R^{x} für C₁-C₄-Alkyl steht, in an sich bekannter Weise zum Alkohol der Formel XIIIa 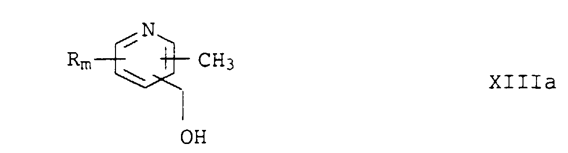 reduziert, XIIIa zum Pyridinaldehyd XIVa 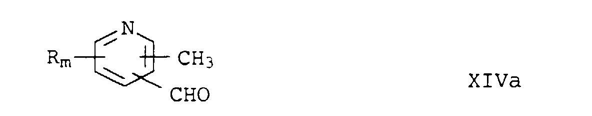 oxidiert, XIVa in an sich bekannter Weise in das Cyanhydrin XVa 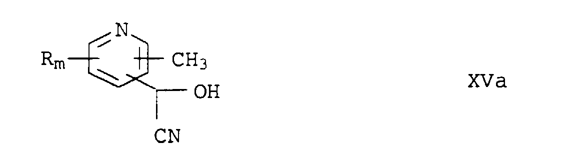 überführt, XVa anschließend zum Mandelsäureester XVIa 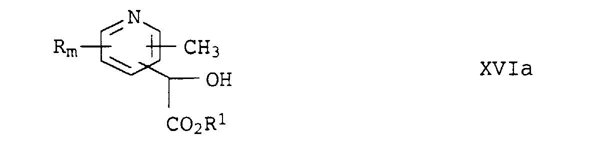 hydrolysiert, XVIa zum α-Ketoester XVIIa 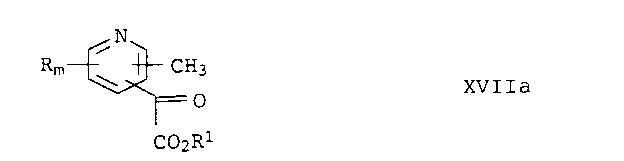 oxidiert und XVIIa anschließend in an sich bekannter Weise entweder
a) mit O-Methylhydroxylamin (H₂NOCH₃) oder einem O-Methylhydroxylammoniumsalz, oder
b) mit einem Ethylen-Wittig- oder -Wittig-Horner Reagens, oder
c) mit einem Methoxy-Wittig- oder -Wittig-Horner Reagens in den entsprechenden Pyridylessigsäureester XVIIIa, XVIIIb bzw. XVIIIc 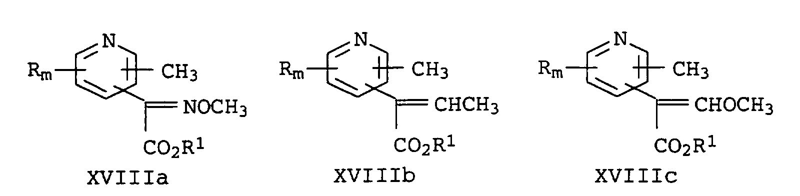
überführt, XVIIIa, XVIIIb bzw. XVIIIc anschließend in an sich bekannter Weise zu den Benzylhalogeniden XIXa, XIXb bzw. XIXc 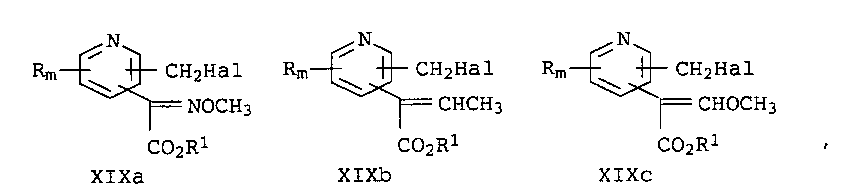 wobei Hal für Chlor oder Brom steht, halogeniert und XIXa, XIXb bzw. XIXc anschließend durch Umsetzung mit einem Hydroxyimin der Formel III gemäß Anspruch 6 in den entsprechenden Pyridylessigsäureester IA überführt.

13. Zwischenprodukte der Formel VIII gemäß Anspruch 8.

14. Zwischenprodukte der Formeln XII, XIII, XIV, XV, XVI und XVII gemäß Anspruch 9.

15. Zwischenprodukte der Formel IIa gemäß Anspruch 11.

16. 2-Methyl-3-formylpyridin-cyanhydrin, (2-Methylpyridyl-3)-α-hydroxyessigsäuremethylester, (2-Methylpyridyl-3)-glyoxylsäuremethylester gemäß Anspruch 12.

17. Zwischenprodukte der Formeln XVIIIa, XVIIIb und XVIIIc gemäß Anspruch 12.

18. Mittel gegen tierische Schädlinge oder Schadpilze, enthaltend übliche Zusatzstoffe und eine wirksame Menge einer Verbindung der Formel I gemäß Anspruch 1.

19. Mittel nach Anspruch 18 zur Bekämpfung tierischer Schädlinge aus der Klasse der Insekten, Spinnentiere oder Nematoden.

20. Verfahren zur Bekämpfung von Insekten, Spinnentieren, Nematoden oder Schadpilzen, jedoch nicht am menschlichen oder tierischen Körper, dadurch gekennzeichnet, daß man die Insekten, Spinnentieren, Nematoden oder Schadpilze, oder die von ihnen freizuhaltenden Pflanzen, Flächen, Materialien oder Räume mit einer wirksamen Menge einer Verbindung der Formel I gemäß Anspruch 1 behandelt.

## Claims

1. A pyridylacetic acid derivative of the formula I 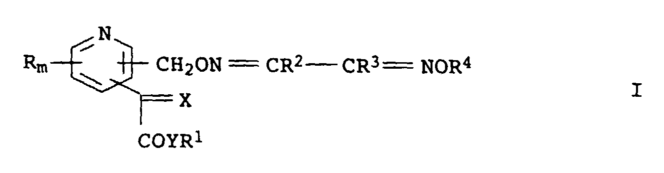 where
X is NOCH₃, CHOCH₃, CHCH₃ or CHCH₂CH₃;
Y is oxygen or NR';
R' is hydrogen or C₁-C₄-alkyl;
R is cyano, nitro, trifluoromethyl, halogen, C₁-C₄-alkyl or C₁-C₄-alkoxy;
m is 0, 1 or 2, and the radicals R may be different when m is 2;
R¹ is hydrogen or C₁-C₄-alkyl;
R² is hydrogen, cyano, nitro, hydroxyl, amino, halogen,
C₁-C₄-alkyl, C₁-C₄-haloalkyl, C₁-C₄-alkoxy, C₁-C₄-haloalkoxy, C₁-C₄-alkylthio, C₁-C₄-alkylamino or di-C₁-C₄-alkylamino;
R³ is hydrogen, cyano, nitro, hydroxyl, amino, halogen,
C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino or N-C₂-C₆-alkynyl-N-C₁-C₆-alkylamino, where the hydrocarbon radicals of these groups may be partly or completely halogenated or may carry from one to three of the following radicals: cyano, nitro, hydroxyl, mercapto, amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio or hetaryl-C₁-C₄-alkylthio, where the cyclic radicals in turn may be partially or completely halogenated and/or may carry from one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR^{a})-Aₙ-R^{b};
C₃-C₆-cycloalkyl, C₃-C₆-cycloalkyloxy, C₃-C₆-cycloalkylthio, C₃-C₆-cycloalkylamino, N-C₃-C₆-cycloalkyl-N-C₁-C₆-alkylamino, C₃-C₆-cycloalkenyl, C₃-C₆-cycloalkenyloxy, C₃-C₆-cycloalkenylthio, C₃-C₆-cycloalkenylamino, N-C₃-C₆-cycloalkenyl-N-C₁-C₆-alkylamino, heterocyclyl, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino or N-hetaryl-N-C₁-C₆-alkylamino, where the cyclic radicals may be partially or completely halogenated or may carry from one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy, C(=NOR^{a})-Aₙ-R^{b} or NR^{f}-CO-D-R⁹;
R⁴ is hydrogen,
C₁-C₁₀-alkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, where these radicals may be partially or completely halogenated or may carry from one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkyloxy, heterocyclyl, heterocyclyloxy, benzyl, benzyloxy, aryl, aryloxy, arylthio, hetaryl, hetaryloxy or hetarylthio, where the cyclic groups in turn may be partially or completely halogenated or may carry from one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR^{a})-Aₙ-R^{b};
C₃-C₆-cycloalkyl, aryl, arylcarbonyl, arylsulfonyl, hetaryl, hetarylcarbonyl or hetarylsulfonyl, where these radicals may be partially or completely halogenated or may carry from one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy, C(=NOR^{a})-Aₙ-R^{b} or NR^{f}-CO-D-R^{g};
A is oxygen, sulfur or nitrogen and the nitrogen carries hydrogen or C₁-C₆-alkyl;
D is a direct bond, oxygen or NR^{h};
n is 0 or 1;
R^{a} and R^{b} are each hydrogen or C₁-C₆-alkyl;
R^{f} is hydrogen, hydroxyl, C₁-C₆-alkyl, C₂-C₆-alkenyl, C₂-C₆-alkynyl, C₁-C₆-alkoxy, C₂-C₆-alkenyloxy, C₂-C₆-alkynyloxy, C₁-C₆-alkoxy-C₁-C₆-alkyl, C₁-C₆-alkoxy-C₁-C₆-alkoxy or C₁-C₆-alkoxycarbonyl;
R^{g}, R^{h} independently of one another, are each hydrogen, C₁-C₆-alkyl, C₂-C₆-alkenyl, C₂-C₆-alkynyl, C₃-C₆-cycloalkyl, C₃-C₆-cycloalkenyl, aryl, aryl-C₁-C₆-alkyl, hetaryl or hetaryl-C₁-C₆-alkyl;
and salts thereof.

2. A compound of the formula I as claimed in claim 1, in which
R² is hydrogen, hydroxyl, cyclopropyl, halogen, C₁-C₄-alkyl, C₁-C₄-alkoxy or C₁-C₄-alkylthio;
R³ is hydrogen, hydroxyl, halogen, cyclopropyl, cyclohexyl, C₁-C₄-alkyl, C₁-C₄-alkoxy, C₁-C₄-alkylthio,
aryl or hetaryl, where these groups may be partially or completely halogenated or may carry from one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl or hetaryloxy;
R⁴ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, where these groups may be partially or completely halogenated or may carry from one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy or hetarylthio, where the aromatic and heteroaromatic radicals in turn may be partially or completely halogenated or may carry from one to three of the following groups; cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR^{a})-Aₙ-R^{b};
aryl, hetaryl, arylcarbonyl, hetarylcarbonyl, arylsulfonyl or hetarylsulfonyl, where these groups may be partially or completely halogenated or may carry from one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR^{a})-Aₙ-R^{b}.

3. A compound of the formula I as claimed in claim 1, in which Y is NR' and X is NOCH₃.

4. A compound of the formula I as claimed in claim 1, in which m is 0.

5. A compound of the formula I as claimed in claim 1, in which R¹ is methyl.

6. A process for the preparation of a compound of the formula I as claimed in claim 1, in which R² is not halogen, wherein a benzyl derivative of the formula II 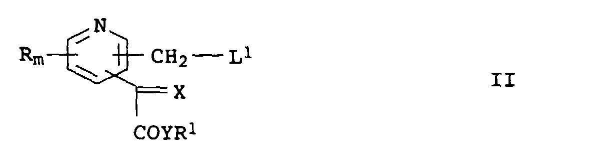 where L¹ is a nucleophilically replaceable leaving group, is reacted in a manner known per se with a hydroximine of the formula III
R⁴ON=C(R³)-C(R²)=NOH III.

7. A process for the preparation of a compound of the formula I as claimed in claim 1, in which R² and R³ are not halogen, wherein a benzyl derivative of the formula II as claimed in claim 1 is reacted in a manner known per se with a dihydroximine of the formula IV
HON=C(R³)-C(R²)=NOH IV
to give a compound of the formula V 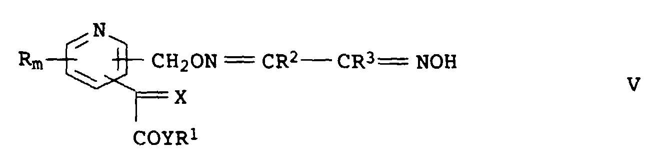 and V is then reacted with a compound of the formula VI
R⁴-L¹ VI
where L² is a nucleophilically replaceable leaving group, to give I.

8. A process for the preparation of a compound of the formula I as claimed in claim 1, in which R² is not halogen, wherein a benzyl derivative of the formula II as claimed in claim 1 is reacted in a manner known per se with a carbonylhydroximine of the formula VII
O=C(R³)-C(R²)=NOH VII
to give a compound of the formula VIII 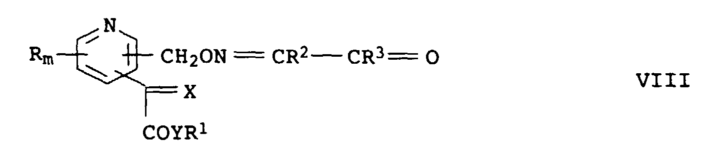 and VIII is then reacted either
a) first with hydroxylamine or its salt and then with a compound of the formula VI (R⁴-L²) as claimed in claim 7 or
b) with a hydroxylamine or a hydroxylammonium salt of the formula IXa or IXb, respectively 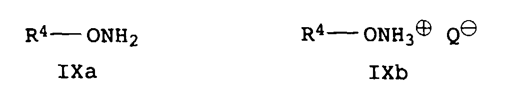 where Q^{⊖} is an anion of an acid,
to give I.

9. A process for the preparation of a compound I as claimed in claim 1, in which Y is oxygen (IA), wherein a methylpyridinecarboxylate of the formula X 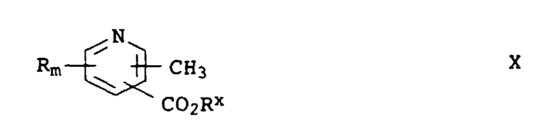 where R^{x} is C₁-C₄-alkyl, is converted in a manner known per se into the corresponding methylenepyridinecarboxylate of the formula XI 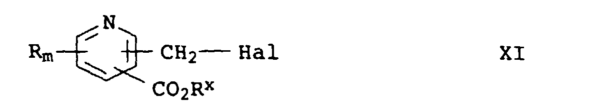 XI is then converted into the corresponding pyridinecarboxylate of the formula XII by reaction with a hydroximine of the formula III as claimed in claim 6, 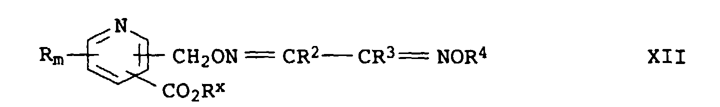 XII is reduced to the alcohol XIII 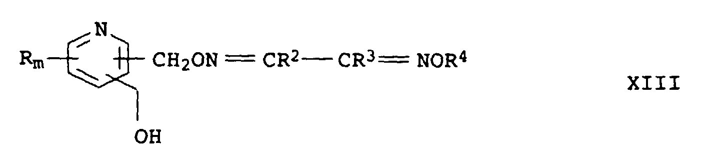 XIII is oxidized to the pyridinealdehyde XIV 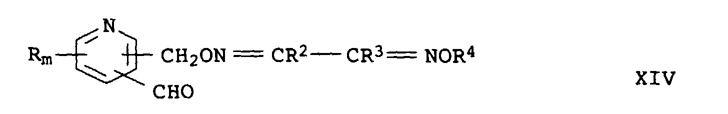 XIV is converted in a manner known per se into the cyanhydrin XV 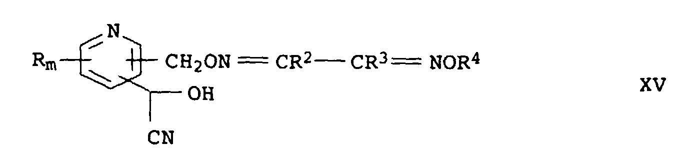 XV is then hydrolyzed to the corresponding mandelate XVI 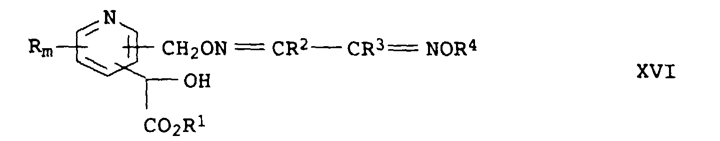 XVI is oxidized to the α-keto ester XVII 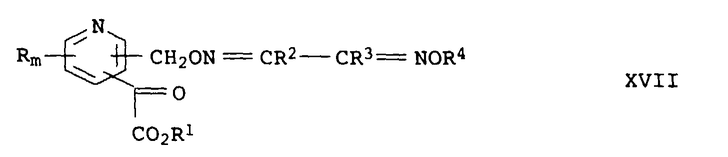 and XVII is then converted in a manner known per se either
a) with O-methylhydroxylamine (H₂NOCH₃) or an O-methylhydroxylammonium salt, or
b) with an ethylene-Wittig or -Wittig-Horner reagent, or
c) with a methoxy-Wittig or -Wittig-Horner reagent
into the corresponding pyridylacetate IA.

10. A process for the preparation of a compound I as claimed in claim 1, in which Y is NR' (IB), wherein the corresponding pyridylacetate of the formula IA as claimed in claim 9 is reacted in a manner known per se with an amine of the formula XVIII
HNR'R¹ XVIII,

11. A process for the preparation of a compound I as claimed in claim 1, wherein a hydroxylamine derivative of the formula IIa 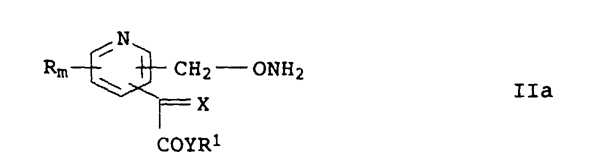 is reacted in a manner known per se with a carbonyl compound of the formula VIIa
O=C(R²)-C(R³)=NOH⁴ VIIa.

12. A process for the preparation of a compound I as claimed in claim 1, in which Y is oxygen (IA), wherein a methylpyridinecarboxylate of the formula X 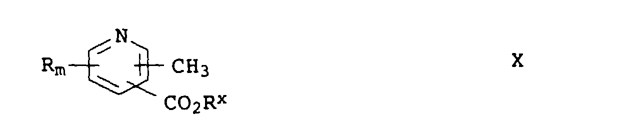 where R^{x} is C₁-C₄-alkyl, is reduced in a manner known per se to the alcohol of the formula XIIIa 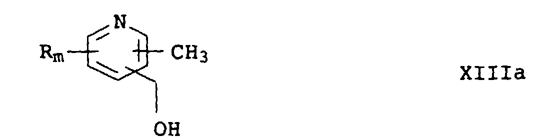 XIIIa is oxidized to the pyridinealdehyd XIVa 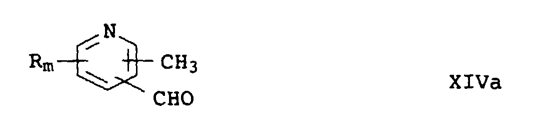 XIVa is converted in a manner known per se into the cyanhydrin XVa 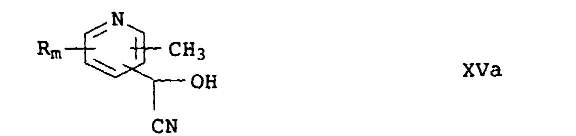 XVa is then hydrolyzed to the mandelate XVIa 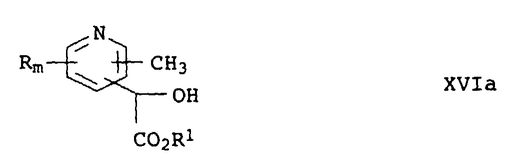 XVIa is oxidized to the α-keto ester XVIIa 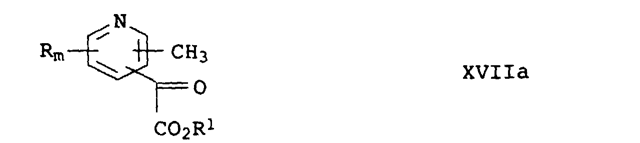 and XVIIa is then converted in a manner known per se either
a) with O-methylhydroxylamine (H₂NOCH₃) or an O-methylhydroxylammonium salt, or
b) with an ethylene-Wittig or -Wittig-Horner reagent, or
c) with a methoxy-Wittig or -Wittig-Horner reagent
into the corresponding pyridylacetate XVIIIa, XVIIIb or XVIIIc, respectively 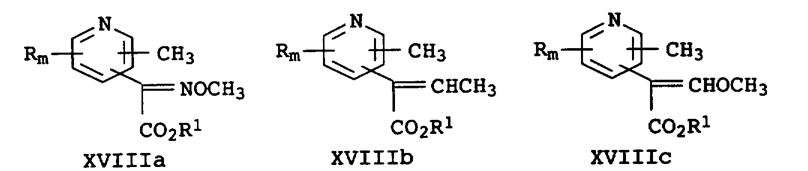 XVIIIa, XVIIIb or XVIIIc is then halogenated in a manner known per se to give the benzyl halide XIXa, XIXb or XIXc, respectively 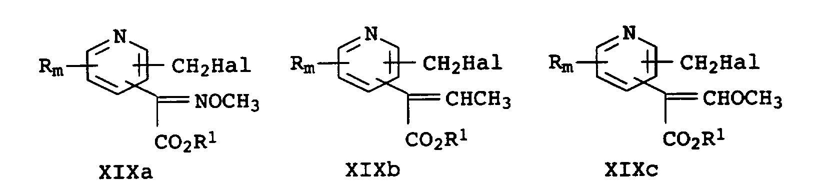 where Hal is chlorine or bromine, and XIXa, XIXb or XIXc is then converted into the corresponding pyridylacetate IA by reaction with a hydroximine of the formula III as claimed in claim 6.

13. An intermediate of the formula VIII as claimed in claim 8.

14. An intermediate of the formula XII, XIII, XIV, XV, XVI or XVII as claimed in claim 9.

15. An intermediate of the formula IIa as claimed in claim 11.

16. 2-Methyl-3-formylpyridinecyanohydrin, methyl (2-methylpyrid-3-yl)-α-hydroxyacetate, methyl (2-methylpyrid-3-yl)glyoxylate as claimed in claim 12.

17. An intermediate of the formula XVIIIa, XVIIIb or XVIIIc as claimed in claim 12.

18. A pesticide or fungicide containing conventional additives and an effective amount of a compound of the formula I as claimed in claim 1.

19. A pesticide as claimed in claim 18 for controlling animal pests from the class consisting of the insects, arachnids or nematodes.

20. A method for controlling insects, arachnids, nematodes or harmful fungi, but not on the human or animal body, wherein the insects, arachnids, nematodes or harmful fungi, or the plants, surfaces, materials or spaces to be kept free from them are treated with an effective amount of a compound of the formula I as claimed in claim 1.

## Revendications

1. Dérivés d'acide pyridylacétique de formule I 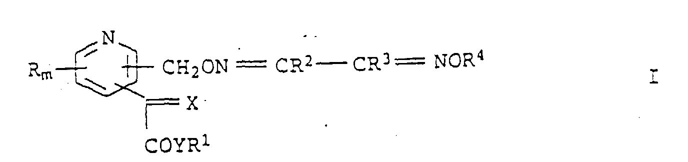 dans laquelle les substituants et l'indice ont la signification suivante:
X NOCH₃, CHOCH₃, CHCH₃ et CHCH₂CH₃;
Y oxygène ou NR';
R' hydrogène ou alkyle en C₁-C₄;
R cyano, nitro, trifluorométhyle, halogéno, alkyle en C₁-C₄ et alcoxy en C₁-C₄;
m 0, 1 ou 2, tandis que les restes R peuvent être différents lorsque m vaut 2;
R¹ hydrogène et alkyle en C₁-C₄;
R² hydrogène, cyano, nitro, hydroxy, amino, halogéno,
alkyle en C₁-C₄, halogénoalkyle en C₁-C₄, alcoxy en C₁-C₄, halogénoalcoxy en C₁-C₄, alkylthio en C₁-C₄, alkylamino en C₁-C₄ ou dialkyl(C₁-C₄)amino;
R³ hydrogène, cyano, nitro, hydroxy, amino, halogéno,
alkyle en C₁-C₆, alcoxy en C₁-C₆, alkylthio en C₁-C₆, alkylamino en C₁-C₆, dialkyl(C₁-C₆)amino, alcényle en C₂-C₆, alcényloxy en C₂-C₆, alcénylthio en C₂-C₆, alcénylamino en C₂-C₆, N-alcényl(C₂-C₆)-N-alkyl(C₁-C₆)amino, alcynyle en C₂-C₆, alcynyloxy en C₂-C₆, alcynylthio en C₂-C₆, alcynylamino en C₂-C₆, N-alcynyl(C₂-C₆)-N-alkyl(C₁-C₆)amino, tandis que les restes hydrocarbonés de ces groupes peuvent être partiellement ou totalement halogénés ou peuvent porter un à trois des restes suivants: cyano, nitro, hydroxy, mercapto, amino, carboxyle, amino-carbonyle, aminothiocarbonyle, halogéno, alkyl(C₁-C₆)aminocarbonyle, dialkyl(C₁-C₆)aminocarbonyle, alkyl(C₁-C₆)aminothiocarbonyle, dialkyl(C₁-C₆)-aminothiocarbonyle, alkyl(C₁-C₆)-sulfonyle, alkyl-(C₁-C₆)sulfoxyle, alcoxy en C₁-C₆, halogénoalcoxy en C₁-C₆, alcoxy(C₁-C₆)-carbonyle, alkylthio en C₁-C₆, alkylamino en C₁-C₆, dialkyl(C₁-C₆)amino, alcényloxy en C₂-C₆, cycloalkyle en C₃-C₆, cycloalkyloxy en C₃-C₆, hétérocyclyle, hétérocyclyloxy, aryle, aryloxy, arylalcoxy(C₁-C₄), arylthio, aryl-alkyl(C₁-C₄)-thio, hétaryle, hétaryloxy, hétaryl-alcoxy-(C₁-C₄), hétarylthio, hétaryl-alkyl(C₁-C₄)thio, tandis que les restes cycliques peuvent à leur tour être partiellement ou totalement halogénés et/ou peuvent porter un à trois des groupes suivants: cyano, nitro, hydroxy, mercapto, amino, carboxyle, aminocarbonyle, aminothio-carbonyle, alkyl en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl(C₁-C₆)amino-carbonyle, alkyl(C₁-C₆)-aminothiocarbonyle, dialkyl(C₁-C₆)aminothio-carbonyle, alcényle en C₂-C₆, alcényloxy en C₂-C₆, benzyle, benzyloxy, aryle, aryloxy, arylthio, hétaryle, hétaryloxy, hétarylthio et C(=NOR^{a})-Aₙ-R^{b};
cycloalkyle en C₃-C₆, cycloalkyloxy en C₃-C₆, cycloalkylthio en C₃-C₆, cycloalkylamino en C₃-C₆, N-cycloalkyl(C₃-C₆)-N-alkyl(C₁-C₆)amino, cyclo-alcényle en C₃-C₆, cycloalcényloxy en C₃-C₆, cycloalcénylthio en C₃-C₆, cycloalcényl-amino en C₃-C₆, N-cycloalkyl(C₃-C₆)-N-alkyl(C₁-C₆)amino, hétérocyclyle, hétérocyclyl-oxy, hétérocyclylthio, hétérocyclylamino, N-hétérocyclyl-N-alkyl(C₁-C₆)-amino, aryle, aryloxy, arylthio, arylamino, N-aryl-N-alkyl(C₁-C₆)amino, hétaryle, hétaryloxy, hétarylthio, hétarylamino, N-hétaryl-N-alkyl(C₁-C₆)amino, tandis que les restes cycliques peuvent être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, cycloaklyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl(C₁-C₆)aminocarbonyle, alkyl(C₁-C₆)amino-thiocarbonyle, dialkyl(C₁-C₆)aminothiocarbonyle, alcényle en C₂-C₆, alcényloxy en C₂-C₆, benzyle, benzyloxy, aryle, aryloxy, hétaryle, hétaryloxy, C(=NOR^{a})-Aₙ-R^{b} ou NR^{f}-CO-D-R^{g};
R⁴ hydrogène,
alkyle en (C₁-C₁₀), alcényle en C₂-C₁₀, alcynyle en C₂-C₁₀, alkyl(C₁-C₁₀)carbonyle, alcényl-(C₂-C₁₀)-carbonyle, alcynyl(C₃-C₁₀)carbonyle ou alkylsulfonyle en C₁-C₁₀, tandis que ces restes peuvent être partiellement ou totalement halogénés ou peuvent porter un à trois des groupes suivants: cyano, nitro, hydroxy, mercapto, amino, carbonyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl(C₁-C₆)amino-carbonyle, alkyl(C₁-C₆)aminothiocarbonyle, dialkyl(C₁-C₆)aminothiocarbonyle, alcényle en C₂-C₆, alcényloxy en C₂-C₆, cycloalkyle en C₃-C₆, cycloalkyloxy en C₃-C₆, hétérocyclyle, hétéro-cyclyloxy, benzyle, benzyloxy, aryle, aryloxy, arylthio, hétaryle, hétaryloxy et hétarylthio, tandis que les groupes cycliques peuvent à leur tour être partiellement ou totalement halogénés ou peuvent porter un à trois des groupes suivants: cyano, nitro, hydroxy, mercapto, amino, carboxyle, amino-carbonyle, aminothiocarbonyle, halogéno, alkyle en C₁-C₆, halogénoalkyle en C₁-C₆, alkyl-sulfonyle en C₁-C₆, alkylsulfoxyle en C₁-C₆, cycloalkyle en C₃-C₆, alcoxy en C₁-C₆, halogéno-alcoxy en C₁-C₆, alkyl(C₁-C₆)oxycarbonyle, alkylthio en C₁-C₆, alkylamino en C₁-C₆, dialkyl(C₁-C₆)amino, alkyl(C₁-C₆)aminocarbonyle, dialkyl(C₁-C₆)aminocarbonyle, alkyl(C₁-C₆)aminothiocarbonyle, dialkyl(C₁-C₆)aminothiocarbonyle, alcényle en C₂-C₆, alcényloxy en C₂-C₆, benzyle, benzyloxy, aryle, aryloxy, arylthio, hétaryle, hétaryloxy, hétarylthio ou C(=NOR^{a})-Aₙ-R^{b};
cycloalkyle en C₃-C₆, aryle, arylcarbonyle, arylsulfonyle, hétaryle, hétarylcarbonyle ou hétarylsulfonyle, tandis que ces restes peuvent être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkyl(C₁-C₆)carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alkyl(C₁-C₆)oxycarbonyle, alkylthio en C₁-C₆, alkylamino en C₁-C₆, dialkyl(C₁-C₆)amino, alkyl-(C₁-C₆)aminocarbonyle, dialkyl(C₁-C₆)-aminocarbonyle, alkyl(C₁-C₆)aminothiocarbonyle, dialkyl(C₁-C₆)aminothiocarbonyle, alcényle en C₂-C₆, alcényloxy en C₂-C₆, benzyle, benzyloxy, aryle, aryloxy, hétaryle, hétaryloxy, C(=NOR^{a})-Aₙ-R^{b} ou NR^{f}-CO-D-R^{g};
tandis que
A désigne l'oxygène, le soufre ou l'azote et tandis que l'azote porte de l'hydrogène ou un alkyle en C₁-C₆;
D une liaison directe, l'oxygène ou NR^{h};
n vaut 0 ou 1;
R^{a} désigne l'hydrogène ou alkyle en C₁-C₆, et
R^{b} représente l'hydrogène ou alkyle en C₁-C₆,
R^{f} désigne l'hydrogène, hydroxy, alkyle en C₁-C₆, alcényle en C₂-C₆, alcynyle en C₂-C₆, alcoxy en C₁-C₆, alcényloxy en C₂-C₆, alcynyloxy en C₂-C₆, alcoxy-(C₁-C₆)alkyle(C₁-C₆), alcoxy(C₁-C₆)-alcoxy(C₁-C₆) et alcoxy(C₁-C₆)carbonyle;
R^{g}, R^{h}, indépendamment l'un de l'autre, hydrogène, alkyle en C₁-C₆, alcényle en C₂-C₆, alcynyle en C₂-C₆, cycloalkyle en C₃-C₆, cycloalcényle en C₃-C₆, aryle, aryl-alkyle(C₁-C₆), hétaryle et hétaryl-alkyle(C₁-C₆);
ainsi que leurs sels.

2. Composés de formule I selon la revendication 1, dans lesquels R², R³ et R⁴ ont la signification suivante:
R² hydrogène, hydroxy, cyclopropyle, halogéno, alkyle en C₁-C₄, alcoxy en C₁-C₄ ou alkylthio en C₁-C₄;
R³ hydrogène, hydroxy, halogéno, cyclopropyle, cyclohexyle, alkyle en C₁-C₄, alcoxy en C₁-C₄, alkylthio en C₁-C₄,
aryle ou hétaryle, tandis que ces groupes peuvent être partiellement ou totalement halogénés ou peuvent porter un à 3 des groupes suivants: cyano, nitro, hydroxy, mercapto, amino, carboxyle, amino-carbonyle, aminothio-carbonyle, halogéno, alkyle en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkyl-amino en C₁-C₆, dialkyl(C₁-C₆)amino, alkyl(C₁-C₆)-aminocarbonyle, dialkyl(C₁-C₆)aminocarbonyle, alkyl(C₁-C₆)aminothiocarbonyle, dialkyl(C₁-C₆)-aminothiocarbonyle, alcényle en C₂-C₆, alcényl-oxy en C₂-C₆, benzyle, benzyloxy, aryle, aryloxy, hétaryle et hétaryloxy;
R⁴ hydrogène,
alkyle en C₁-C₁₀, cycloalkyle en C₃-C₆, alcényle en C₂-C₁₀, alcynyle en C₂-C₁₀, alkyl(C₁-C₁₀)-carbonyle, alcényl(C₂-C₁₀)carbonyle, alcynyl-(C₃-C₁₀)carbonyle ou alkylsulfonyle en C₁-C₁₀, tandis que ces groupes peuvent être partiellement ou totalement halogénés ou peuvent porter un à 3 des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)-amino-carbonyle, dialkyl(C₁-C₆)aminocarbonyle, alkyl-(C₁-C₆)aminothiocarbonyle, dialkyl(C₁-C₆)-aminothiocarbonyle, alcényle en C₂-C₆, alcényloxy en C₂-C₆, benzyle, benzyloxy, aryle, aryloxy, arylthio, hétaryle, hétaryloxy et hétarylthio, tandis que les restes aromatiques et hétéro-aromatiques peuvent à leur tour être partiellement ou totalement halogénés et/ou peuvent porter un à trois des groupes suivants: cyano, nitro, hydroxy, mercapto, amino, carboxyle, aminocarbonyle, amino-thiocarbonyle, halogéno, alkyle en C₁-C₆, halogénoalkyle en C₁-C₆, alkylsulfonyle en C₁-C₆, alkylsylfoxyle en C₁-C₆, cycloalkyle en C₃-C₆, alcoxy en C₁-C₆, halogénoalcoxy en C₁-C₆, alkyl(C₁-C₆)oxy-carbonyle, alkylthio en C₁-C₆, alkylamino en C₁-C₆, dialkyl(C₁-C₆)amino, alkyl(C₁-C₆)aminocarbonyle, dialkyl(C₁-C₆)amino-carbonyle, alkyl(C₁-C₆)aminothiocarbonyle, dialkyl(C₁-C₆)aminothiocarbonyle, alcényle en C₂-C₆, alcényloxy en C₂-C₆, benzyle, benzyloxy, aryle, aryloxy, arylthio, hétaryle, hétaryloxy, hétarylthio ou C(=NOR^{a})-Aₙ-R^{b};
aryle, hétaryle, arylcarbonyle, hétaryl-carbonyle, arylsulfonyle ou hétarylsulfonyle, tandis que ces groupes peuvent être partiellement ou totalement halogénés ou peuvent porter un à 3 des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkyl(C₁-C₆)carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl(C₁-C₆)amino-carbonyle, alkyl(C₁-C₆)aminothiocarbonyle, di-alkyl(C₁-C₆)aminothiocarbonyle, alcényle en C₂-C₆, alcényloxy en C₂-C₆, benzyle, benzyloxy, aryle, aryloxy, hétaryle, hétaryloxy ou C(=NOR^{a})-Aₙ-R^{b}.

3. Composés de formule I selon la revendication 1, dans lesquels Y désigne NR' et X représente NOCH₃.

4. Composés de formule I selon la revendication 1, dans lesquels m vaut 0.

5. Composés de formule I selon la revendication 1, dans lesquels R¹ représente un méthyle.

6. Procédé pour la préparation des composés de formule I selon la revendication 1, dans lesquels R² ne désigne pas un halogène, caractérisé par le fait qu'on fait réagir un dérivé benzylique de formule II 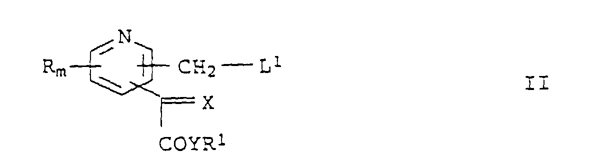 dans laquelle L¹ représente un groupe éliminable échangeable par voie nucléophile, d'une manière connue en soi avec une hydroxyimine de formule III
R⁴ON=C(R³)-C(R²)=NOH III

7. Procédé pour la préparation des composés de formule I selon la revendication 1, dans lesquels R² et R³ ne désignent pas un halogène, caractérisé par le fait qu'on fait réagir un dérivé benzylique de formule II selon la revendication 1, de manière connue en soi avec une dihydroxyimine de formule IV
HON=C(R³)-C(R²)=NOH IV
pour former un composé de formule V 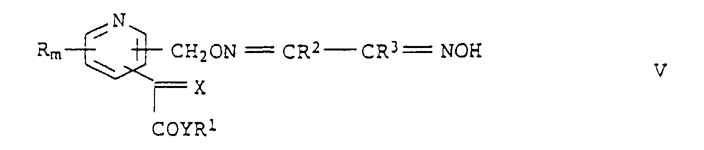 et on convertit ensuite V en I avec un composé de formule VI
R⁴-L¹ VI
dans laquelle L² représente un groupe éliminable échangeable par voie nucléophile.

8. Procédé pour la préparation des composés de formule I selon la revendication 1, dans lesquels R² ne représente pas un halogène, caractérisé par le fait qu'on fait réagir un dérivé benzylique de formule II selon la revendication 1, de manière connue en soi avec une carbonylhydroxyimine de formule VII
O=C(R³)-C(R²)=NOH VII
pour former un composé de formule VIII 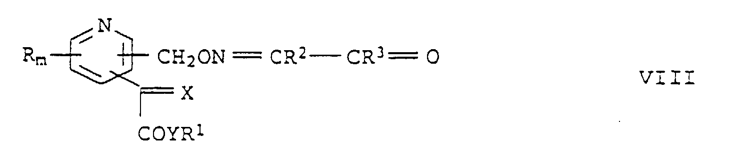 ensuite on convertit VIII
a) d'abord avec de l'hydroxylamine ou un sel de celle-ci et ensuite avec un composé de formule VI (R⁴-L²) selon la revendication 7
ou
b) avec une hydroxylamine ou un sel d'hydroxyl-ammonium de formule IXa ou IXb 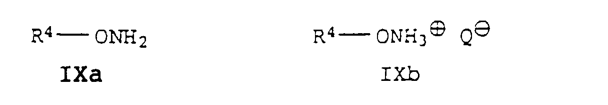 où Q^{⊖} représente l'anion d'un acide.

9. Procédé pour la préparation des composés I selon la revendication 1, dans lesquels Y désigne l'oxygène (IA), caractérisé par le fait qu'on convertit un ester d'acide méthylpyridinecarboxylique de formule générale X 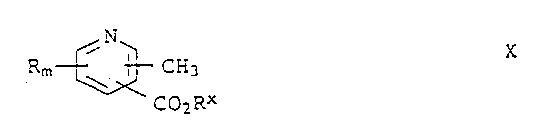 dans laquelle R^{x} représente un alkyle en C₁-C₄, d'une manière connue en soi en l'ester d'acide méthylènepyridinecarboxylique correspondant de formule générale XI 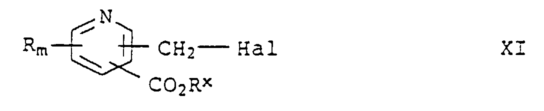 on transforme ensuite XI par réaction avec une hydroxyimine de formule III selon la revendication 6 en l'ester d'acide pyridinecarboxylique correspondant de formule générale XII 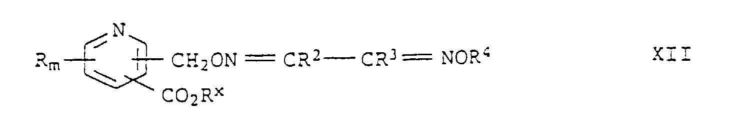 on réduit XII en l'alcool XIII 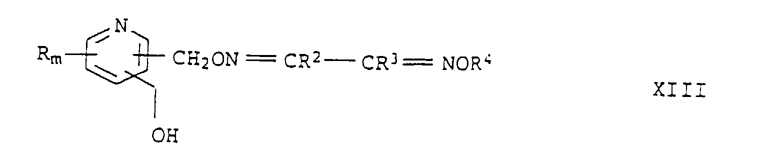 on oxyde XIII en l'aldéhyde de pyridine XIV 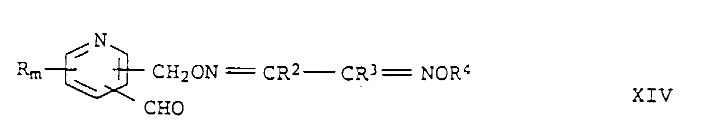 on convertit XIV, d'une manière connue en soi en la cyanhydrine XV 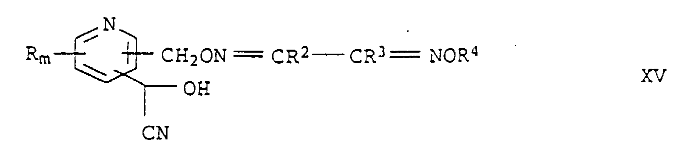 on hydrolyse ensuite XV pour former l'ester d'acide mandélique correspondant XVI 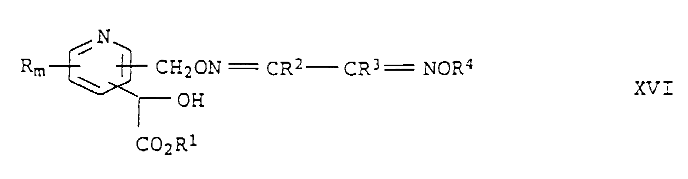 on oxyde XVI pour former l'α-cétoester XVII 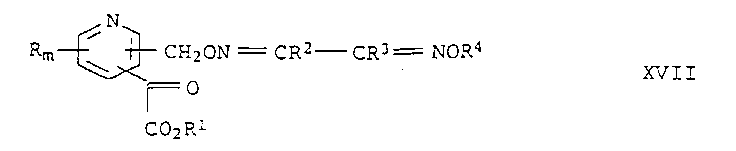 et on convertit ensuite XVII en l'ester d'acide pyridylacétique IA correspondant, d'une manière connue en soi, soit
a) avec de l'O-méthylhydroxylamine (H₂NOCH₃) ou avec un sel d'O-méthylhydroxylammonium, soit
b) avec un réactif de Wittig ou de Wittig-Horner éthyléné, soit
c) avec un réactif de Wittig ou de Wittig-Horner méthoxylé.

10. Procédé pour la préparation des composés I selon la revendication 1, dans lesquels Y désigne NR' (IB), caractérisé par le fait qu'on fait réagir l'ester d'acide pyridylacétique correspondant de formule IA selon la revendication 9, d'une manière connue en soi avec une amine de formule XVIII
HNR'R¹ XVIII

11. Procédé pour la préparation des composés I selon la revendication 1, caractérisé par le fait qu'on fait réagir un dérivé d'hydroxylamine de formule IIa 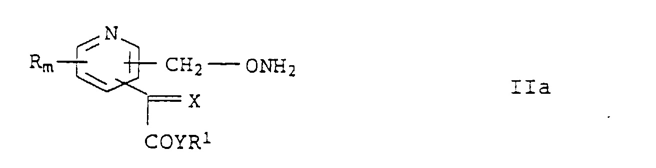 d'une manière connue en soi avec un composé carbonyle de formule VIIa
O=C(R²)-C(R³)=NOH⁴ VIIa

12. Procédé pour la préparation des composés I selon la revendication 1, dans lesquels Y représente l'oxygène
(IA), caractérisé par le fait qu'on réduit un ester d'acide méthylpyridine carboxylique de formule X 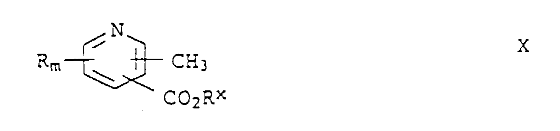 dans laquelle R^{x} désigne un alkyle en C₁-C₄, d'une manière connue en soi pour former l'alcool de formule XIIIa 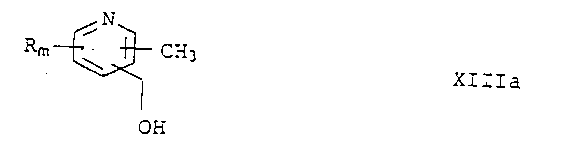 on oxyde XIIIa en l'aldéhyde de pyridine XIVa 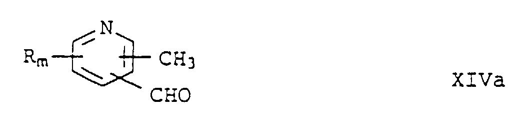 on convertit XIVa, d'une manière connue en soi en la cyanhydrine XVa 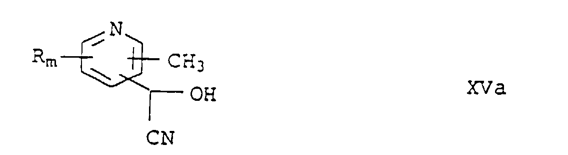 on hydrolyse ensuite XVa en l'ester d'acide mandélique XVIa 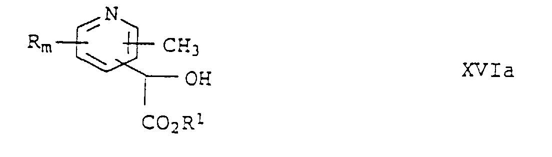 on oxyde XVIa en l'α-cétoester XVIIa 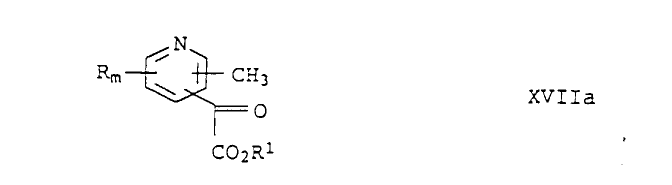 et on convertit ensuite XVIIa, d'une manière connue en soi, soit
a) avec de l'O-méthylhydroxylamine (H₂NOCH₃) ou avec un sel d'O-méthylhydroxylammonium, soit
b) avec un réactif de Wittig ou de Wittig-Horner éthyléné, soit
c) avec un réactif de Wittig ou de Wittig-Horner méthoxylé,
en l'ester d'acide pyridylacétique correspondant XVIIIa, XVIIIb ou XVIIIc 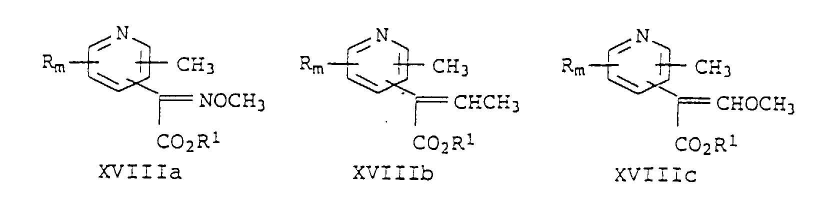 on halogène ensuite XVIIIa, XVIIIb ou XVIIIc, d'une manière connue en soi en les halogénures de benzyle XIXa, XIXb ou XIXc 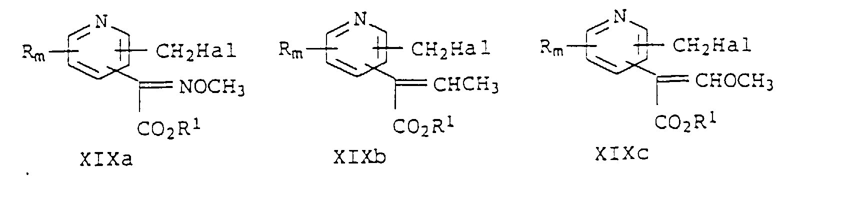 où Hal représente le chlore ou le brome,
et on convertit ensuite XIXa, XIXb ou XIXc par réaction avec une hydroxyimine de formule III selon la revendication 6 en l'ester d'acide pyridylacétique IA correspondant.

13. Produits intermédiaires de formule VIII selon la revendication 8.

14. Produits intermédiaires de formules XII, XIII, XIV, XV, XVI et XVII selon la revendication 9.

15. Produits intermédiaires de formules IIa selon la revendication 11.

16. 2-méthyl-3-formylpyridine-cyanhydrine, ester méthylique d'acide (2-méthylpyridyl-3)-a-hydroxyacétique, ester méthylique d'acide (2-méthylpyridyl-3)-glyoxalique, selon la revendication 12.

17. Produits intermédiaires de formules XVIIIa, XVIIIb et XVIIIc selon la revendication 12.

18. Produit contre les parasites des animaux ou les champignons nuisibles, contenant des additifs classiques et une quantité efficace d'un composé de formule I selon la revendication 1.

19. Produit selon la revendication 18 pour la lutte contre les parasites des animaux de la classe des insectes, des arachnides ou des nématodes.

20. Procédé pour la lutte contre les insectes, les arachnides, les nématodes ou les champignons nuisibles, mais pas sur les corps des hommes ou des animaux, caractérisé par le fait qu'on traite les insectes, arachnides, nématodes ou champignons nuisibles, ou les plantes, surfaces, matériaux ou espaces à débarrasser de ceux-ci, avec une quantité efficace d'un composé de formule I selon la revendication 1.
